# EUROPÄISCHE PATENTSCHRIFT

(11) **EP 3 115 616 B1**
(45) Veröffentlichungstag und Bekanntmachung des Hinweises auf die Patenterteilung: **07.09.2022**
(21) Anmeldenummer: 16172161.8
(22) Anmeldetag: 31.05.2016
(51) Int. Cl.: H02K 21/02, H02K 19/10, H02K 7/09, F16C 32/04

(54) **ELEKTROMAGNETISCHER DREHANTRIEB**
ELECTROMAGNETIC ROTARY DRIVE
ENTRAINEMENT ROTATIF ELECTROMAGNETIQUE

(30) Priorität: 06.07.2015 EP 15175531
(43) Veröffentlichungstag der Anmeldung: 11.01.2017
(60) Teilanmeldung: 22161796.2
(73) Patentinhaber: Levitronix GmbH, 8005 Zürich (CH)
(72) Erfinder: Schöb, Reto Dr., 8964 Rudolfstetten (CH); Hugel, Jörg Prof. Dr., 8008 Zürich (CH); Holenstein, Thomas, 5222 Umiken (CH)
(74) Vertreter: IPS Irsch AG

(56) Entgegenhaltungen:
- EP-A1- 0 899 855
- EP-A1- 1 814 212
- JP-A- 2000 184 655
- JP-A- 2014 209 832
- US-A1- 2009 121 571
- US-A1- 2013 207 504

## Beschreibung

### Elektromagnetischer Drehantrieb

Die Erfindung betrifft einen elektromagnetischen Drehantrieb gemäss dem Oberbegriff des unabhängigen Patentanspruchs.

Es sind elektromagnetische Drehantriebe bekannt, die nach dem Prinzip des lagerlosen Motors ausgestaltet sind und betrieben werden. Mit dem Begriff lagerloser Motor ist dabei ein elektromagnetischer Drehantrieb gemeint, bei welchem der Rotor vollkommen magnetisch bezüglich des Stators gelagert ist, wobei keine separaten magnetischen Lager vorgesehen sind. Der Stator ist dazu als Lager- und Antriebsstator ausgestaltet, der sowohl Stator des elektrischen Antriebs als auch Stator der magnetischen Lagerung ist. Mit den elektrischen Wicklungen des Stators lässt sich ein magnetisches Drehfeld erzeugen, welches zum einen ein Drehmoment auf den Rotor ausübt, das dessen Rotation bewirkt, und welches zum anderen eine beliebig einstellbare Querkraft auf den Rotor ausübt, sodass dessen radiale Position aktiv steuerbar bzw. regelbar ist. Das Nichtvorhandensein eines separaten magnetischen Lagers bei vollständiger magnetischer Lagerung des Rotors ist die Eigenschaft, welcher der lagerlose Motor seinen Namen verdankt.

Der lagerlose Motor ist dem Fachmann mittlerweile hinlänglich bekannt, und wird für zahlreiche verschiedene Anwendungen eingesetzt. Grundlegende Beschreibungen finden sich beispielsweise in der EP0899855, JP2000184655, WO9859407, EP-A-0 860 046 und in der EP-A-0 819 330. Aufgrund der Abwesenheit von mechanischen Lagern eignet sich der lagerlose Motor insbesondere für Pump- und Mischvorrichtungen, mit denen sehr empfindliche Substanzen gefördert werden, beispielsweise Blutpumpen, oder bei denen sehr hohe Anforderungen an die Reinheit gestellt werden, beispielsweise in der pharmazeutischen Industrie oder in der biotechnologischen Industrie, oder mit denen abrasive Substanzen gefördert werden, welche mechanische Lager sehr schnell zerstören würden, beispielsweise Pumpen oder Mischer für Slurry in der Halbleiterindustrie. Auch werden lagerlose Motoren in der Halbleiterfertigung zum Tragen und Rotieren von Wafern verwendet, beispielsweise, wenn diese mit Photolack oder anderen Substanzen beschichtet bzw. behandelt werden.

Ein weiterer Vorteil des Prinzips des lagerlosen Motors bei Pump- oder Mischanwendungen ergibt sich bei der Ausgestaltung des Rotors als Integralrotor, der sowohl der Rotor des elektromagnetischen Antriebs ist, als auch der Rotor der Pumpe oder des Mischers. Neben der berührungslosen magnetischen Lagerung resultiert hier der Vorteil einer sehr kompakten und platzsparenden Ausgestaltung.

Zudem erlaubt das Prinzip des lagerlosen Motors auch Ausgestaltungen, bei denen der Rotor sehr leicht vom Stator trennbar ist. Dies ist ein sehr grosser Vorteil, weil damit beispielsweise der Rotor als Einmalteil für den Einmalgebrauch ausgestaltet werden kann. Solche Einmalanwendungen ersetzen heute häufig Prozesse, bei denen früher aufgrund der sehr hohen Reinheitsanforderungen alle diejenigen Komponenten, welche im Prozess mit den zu behandelnden Substanzen in Kontakt kommen, aufwändig gereinigt und sterilisiert werden müssen, beispielsweise mittels Dampfsterilisierung. Bei der Ausgestaltung für den Einmalgebrauch werden diejenigen Komponenten, welche mit den zu behandelnden Substanzen in Kontakt kommen, nur genau einmal verwendet und dann bei der nächsten Anwendung durch neue, das heisst ungebrauchte, Einmalteile ersetzt.

Als Beispiele seien hier die Pharmaindustrie und die biotechnologische Industrie genannt. Hier werden häufig Lösungen und Suspensionen hergestellt, die eine sorgfältige Durchmischung oder Förderung von Substanzen verlangen.

In der Pharmaindustrie müssen beispielsweise bei der Herstellung von pharmazeutisch wirksamen Substanzen höchste Ansprüche an die Reinheit gestellt werden, oft müssen die mit den Substanzen in Kontakt kommenden Komponenten sogar steril sein. Ähnliche Anforderungen ergeben sich auch in der Biotechnologie, beispielsweise bei der Herstellung, Behandlung oder Züchtung von biologischen Substanzen, Zellen oder Mikroorganismen, wo ein extrem hohes Mass an Reinheit gewährleistet sein muss, um die Brauchbarkeit des hergestellten Produkts nicht zu gefährden. Als ein weiteres Beispiel seien hier Bioreaktoren genannt, in denen beispielsweise biologische Substitute für Gewebe oder spezielle Zellen oder andere sehr empfindliche Substanzen gezüchtet werden. Auch hier benötigt man Pump- oder Mischvorrichtungen, um beispielsweise eine kontinuierliche Durchmischung der Nährflüssigkeit beziehungsweise deren kontinuierliche Zirkulation im Mischbehälter zu gewährleisten. Dabei muss eine sehr hohe Reinheit gewährleistet sein, um die Substanzen oder die erzeugten Produkte vor Kontaminationen zu schützen.

Bei solchen Anwendungen setzt sich dann die Pump- oder Mischvorrichtung aus einer Einmalvorrichtung und einer wiederverwendbaren Vorrichtung zusammen. Dabei umfasst die Einmalvorrichtung diejenigen Komponenten, die mit den Substanzen in Kontakt kommen und die als Einmal (single-use) -teile für den Einmalgebrauch ausgestaltet sind. Dies ist beispielsweise der Pump-oder Mischbehälter mit dem darin vorgesehenen Rotor, der dann beispielsweise ein Flügelrad zum Fördern der Substanzen umfasst. Die wiederverwendbare Vorrichtung umfasst diejenigen Komponenten, die dauerhaft also mehrfach verwendet werden, beispielsweise den Stator. Eine solche Vorrichtung ist zum Beispiel in der EP-B-2 065 085 offenbart.

Bei der Ausgestaltung als Einmalteil ist der Pump- oder Mischbehälter häufig als flexibler Kunststoffbeutel bzw. als Plastiksack mit darin enthaltenem Rotor ausgestaltet. Diese Beutel werden oft schon bei der Herstellung oder aber nach dem Verpacken und dem Lagern sterilisiert und dem Kunden in steriler Form in der Verpackung zugestellt.

Bei der Herstellung bzw. der Konzipierung von Einmalteilen für den Einmalgebrauch ist es ein wichtiges Kriterium, dass sie in möglichst einfacher Weise mit der wiederverwendbaren Vorrichtung bzw. deren Komponenten zusammengesetzt werden können. Es ist wünschenswert, dass dieses Zusammensetzen mit möglichst geringem Aufwand, mit wenigen Handgriffen, rasch und vorzugsweise ohne Werkzeug erfolgen kann.

Ein anderer Aspekt ist es, dass diese Einmalteile möglichst wirtschaftlich und kostengünstig gefertigt werden können. Hierbei wird insbesondere auch Wert auf preisgünstige, einfache Ausgangsmaterialien, wie beispielsweise handelsübliche Kunststoffe, gelegt. Auch ein umweltbewusster Umgang sowie eine verantwortungsvolle Nutzung der zur Verfügung stehenden Ressourcen sind wesentliche Aspekte bei der Konzeption von Einwegteilen.

Auch sind solche Ausgestaltungen bekannt, bei denen die gesamte Pump- oder Mischvorrichtung für den Einmalgebrauch ausgestaltet ist.

Ausgehend von diesem Stand der Technik ist es eine Aufgabe der Erfindung, einen anderen elektromagnetischen Drehantrieb bereitzustellen, der nach dem Prinzip des lagerlosen Motors ausgestaltet ist und der sich für eine Vielzahl von Anwendungen einsetzen lässt. Ferner soll der Drehantrieb auch für Anwendungen mit Komponenten für den Einmalgebrauch ausgestaltbar sein.

Der diese Aufgabe lösende Gegenstand der Erfindung ist durch die Merkmale des unabhängigen Patentanspruchs gekennzeichnet.

Erfindungsgemäss wird also ein elektromagnetischer Drehantrieb gemäss Anspruch 1 vorgeschlagen.

Durch die spezielle Ausgestaltung des Stators, der einen Permanentmagneten umfasst, ist es möglich, einen sehr grossen Teil des magnetischen Flusses oder sogar den gesamten magnetischen Fluss im Stator zu generieren. Hierdurch wird es insbesondere möglich, dass der Rotor wenig oder gar nicht zur Generierung des magnetischen Flusses beitragen, sondern diesen nur leiten bzw. führen muss. Somit kann im Rotor auf starke Permanentmagnete bzw. sehr hartmagnetische Stoffe verzichtet werden.

Es ist gemäss dem heutigen Stand der Technik üblich, als Permanentmagnete im Rotor insbesondere Metalle der Seltenen Erden bzw. Verbindungen oder Legierungen dieser Metalle zu verwenden, weil sich mit diesen aufgrund ihrer magnetischen Eigenschaften sehr starke permanentmagnetische Felder erzeugen lassen. Bekannte und häufig verwendete Beispiele dieser Seltenen Erden sind Neodym und Samarium. Solche Metalle stellen jedoch aufgrund ihres vergleichsweise geringen Vorkommens sowie ihrer aufwändigen Gewinnung und Verarbeitung einen erheblichen Kostenfaktor dar. Zudem ist die Entsorgung solcher Permanentmagnete, beispielsweise nach dem Einmalgebrauch auch unter umwelttechnischen Aspekten häufig mit Problemen oder hohem Aufwand verbunden, wodurch zusätzliche Kosten entstehen. Es ist daher unter wirtschaftlichen, Kosten- und Umweltaspekten insbesondere auch bei Einmalanwendungen vorteilhaft, dass es die Erfindung ermöglicht, bei den Rotoren insbesondere auf solche permanentmagnetischen Materialien, die aus Seltenen Erden bestehen oder diese enthalten, verzichten zu können.

Vorzugsweise umfasst der Rotor einen magnetisch wirksamen Kern und ist frei von Permanentmagneten. Durch den vollständigen Verzicht auf Permanentmagnete im Rotor lässt sich dieser besonders einfach, wirtschaftlich und kostengünstig produzieren, was insbesondere auch für eine Ausgestaltung als Einmal-Rotor einen enormen Vorteil darstellt. Im Bereich zwischen dem Stator und dem magnetisch wirksamen Kern des Rotors werden je nach Ausgestaltung häufig verschiedene Ummantelungen, Spalte und Wandungen untergebracht, insbesondere eine Ummantelung des magnetisch wirksamen Kerns, der Fluidspalt, oder ein Spalttopf, welcher den Stator umgibt. Um alle diese Elemente unterzubringen, ist ein Abstand von mindestens 3 Millimetern, besser von 4-6 Millimetern zwischen dem Stator und dem magnetisch wirksamen Kern des Rotors bevorzugt. Da der Rotor des erfindungsgemässen vorzugsweise keine Permanentmagnete aufweisen soll und somit nicht zur magnetischen Durchflutung beitragen kann, muss die gesamte magnetische Durchflutung im Stator erzeugt werden. Für einen Abstand von beispielsweise 3 Millimetern zwischen Stator und dem magnetisch wirksamen Kern des Rotors ist vorzugsweise eine Durchflutung von rund 5000 Ampere erforderlich, um den Rotor wirksam magnetisch lagern und antreiben zu können. Wird der Stator wie üblich allein mit Wicklungen erregt, ist bei vernünftigen Abmessungen eine solch hohe Durchflutung unmöglich im meist engen Bauraum des Stators realisierbar. Erfindungsgemäss werden deshalb im Stator ein oder mehrere Permanentmagnete angebracht, welche einen konstanten Vormagnetisierungsfluss erzeugen. Da sich mit einem konstanten Magnetfluss jedoch weder ein Drehfeld zur Erzeugung eines Drehmoments noch ein regelbarer Magnetfluss zur aktiven magnetischen Lagerung des Rotors erzeugen lassen, werden im Stator zusätzlich Spulen angebracht, durch welche zusätzliche elektromagnetische und damit veränderbare und regelbare Magnetflüsse erzeugt werden. Vorzugsweise werden dabei die elektromagnetischen Flusspfade so geführt, dass diese nicht durch den oder die Permanentmagnete hindurchführen. Die meisten Permanentmagnete, insbesondere Seiten-Erden-Magnete aber auch Ferritmagnete haben eine relative Permeabilität, welche nur unwesentlich über eins liegt. Würden die elektromagnetischen Flusspfade also durch den oder die Permanentmagnet/e hindurch geführt, würde sich der elektromagnetisch wirksame Luftspalt somit um die Bauhöhe der sich im Flusspfad befindlichen Permanentmagnete erhöhen und den Durchflutungsbedarf zusätzlich erhöhen. Es ist somit auch ein wesentlicher Vorteil der Erfindung, dass die permanentmagnetisch erregten Flüsse und die elektromagnetisch erregten Flüsse so geführt werden können, dass diese sich im magnetischen Luftspalt zwischen dem Stator und dem Rotor überlagern, im Bereich der Permanentmagnete aber getrennt geführt werden. Die elektromagnetisch erregten Flüsse sollen vorzugsweise ausser im Bereich der Luftspalte zwischen Rotor und Stator, möglichst durch weichmagnetisches Material wie Eisen oder Silizium-Eisen geführt werden. Durch die Überlagerung der permanentmagnetisch erregten Flüsse und der elektromagnetisch erregten Flüsse im Bereich der Luftspalte zwischen Rotor und Stator können die Luftspaltflüsse so moduliert werden, dass sowohl eine Regelung der radialen Rotorposition als auch die Bildung von tangentialen Kraftkomponenten, welche ein Drehmoment bewirken, ermöglicht wird.

Bezüglich des Stators ist es vorteilhaft, wenn der obere Statorteil oder der untere Statorteil genau drei oder genau vier oder genau sechs obere bzw. untere Pole umfasst. Die Ausgestaltung mit drei Polen hat den Vorteil, dass sie insbesondere bei Ausführungsformen von Rotor und Stator als Aussenläufer an den Polen des Stators besonders viel Platz für die Spulen bzw. die Wicklungen des Stators lässt. Zudem ermöglicht diese Ausgestaltung eine besonders hohe elektromagnetische Durchflutung. Die Ausgestaltung mit vier Polen hat den Vorteil, dass sie eine besonders symmetrische Anordnung der Pole ermöglicht, was steuer- bzw. regelungstechnisch besonders günstig ist. Die Ausgestaltung mit sechs Polen ist vorteilhaft, weil sie eine besonders günstige und homogene Erzeugung des Drehmoments und der Querkraft auf den Rotor ermöglicht.

Es ist bevorzugt, wenn die Anzahl der oberen Pole gleich der Anzahl der unteren Pole ist. Dies ermöglicht einen besonders einfachen Herstellungsprozess und vereinfacht zudem die elektrische Ansteuerung und die Regelung der Vorrichtung.

Eine weitere vorteilhafte Ausgestaltung besteht darin, dass der obere Statorteil und der untere Statorteil bezüglich der Solldrehachse um einen Winkel verdreht zueinander angeordnet sind, sodass in axialer Richtung gesehen die oberen Pole jeweils in einer Lücke zwischen zwei benachbarten unteren Polen angeordnet sind, wobei der Winkel vorzugsweise 360° dividiert durch die Gesamtzahl der oberen und der unteren Pole beträgt. Durch diese Massnahme lässt es sich besonders gut gewährleisten, dass mit dem einen der beiden Statorteile ein Drehmoment auf den Rotor generierbar ist, während gleichzeitig mit dem anderen Statorteil eine resultierende Querkraft in radialer Richtung auf den Rotor erzeugt werden kann.

Bei einer weiteren bevorzugten Ausführungsform ist die Anzahl der oberen Pole und die Anzahl der unteren Pole eine gerade Zahl, wobei die oberen Pole und die unteren Pole so angeordnet sind, dass sie sich in axialer Richtung gesehen überdecken. Mit dieser Massnahme kann der Rotor auch bezüglich Verkippungen gegen die Solldrehachse (zwei Freiheitsgrade) aktiv magnetisch geregelt werden.

Eine weitere bevorzugte Ausführungsform ist dadurch gekennzeichnet, dass der Rotor mindestens ein Flügelrad zum Fördern von Fluiden umfasst. Somit kann der Rotor des elektromagnetischen Drehantriebs gleichzeitig der Rotor einer Pump- oder Mischvorrichtung sein.

Eine andere bevorzugte Ausführungsform ist es, dass mindestens zwei separate Rotoren vorgesehen sind, von denen jeder jeweils berührungslos magnetisch antreibbar ist und jeweils spulenfrei ausgestaltet ist, wobei die Rotoren im Betriebszustand bezüglich der axialen Richtung beabstandet zueinander und koaxial angeordnet sind, sowie mindestens zwei Statoren, von denen jeder als Lager- und Antriebsstator ausgestaltet ist, wobei jeder Stator jeweils einen oberen Statorteil mit einer Mehrzahl von ausgeprägten oberen Polen zum Tragen von oberen Wicklungen umfasst, sowie einen unteren Statorteil mit einer Mehrzahl von ausgeprägten unteren Polen zum Tragen von unteren Wicklungen, wobei der obere Statorteil und der untere Statorteil jedes Stators bezüglich der axialen Richtung beabstandet zueinander angeordnet sind, wobei jeweils zwischen dem oberen Statorteil und dem unteren Statorteil ein Permanentmagnet vorgesehen ist und wobei die beiden Statoren im Betriebszustand bezüglich der axialen Richtung beabstandet zueinander angeordnet sind. Diese Ausführungsform mit mindestens zwei Statoren und mindestens zwei Rotoren ermöglicht beispielsweise bei Misch- oder Pumpvorrichtungen Betriebszustände, bei denen die beiden Rotoren in entgegengesetzter Richtung und/oder mit unterschiedlichen Geschwindigkeiten rotieren. Dabei ist jedem Rotor ein eigener Stator zugeordnet, welcher diesen Rotor berührungslos antreibt und gleichzeitig berührungslos magnetisch lagert.

Eine vorteilhafte Massnahme besteht darin, dass der magnetisch wirksame Kern des Rotors mehrere ausgeprägte Rotorpole aufweist, welche im Betriebszustand den Polen des Stators zugewandt sind. Hierdurch lässt sich eine besonders gute und effiziente Führung des magnetischen Flusses gewährleisten.

Dabei ist es je nach Ausgestaltung eine bevorzugte Massnahme, wenn die Rotorpole derart asymmetrisch ausgestaltet oder angeordnet sind, dass im Betriebszustand Einrastpositionen bezüglich des Stators vermieden werden. Diese Asymmetrie ist besonders geeignet, wenn der Stator eine relativ kleine Anzahl von Polen umfasst, beispielsweise jeweils drei oder vier obere und untere Pole. Zur Realisierung der Asymmetrie gibt es mehrere Möglichkeiten, beispielsweise kann durch die in Umfangsrichtung gemessene Länge der Rotorpole die Symmetrie zwischen den Rotorpolen und den Polen des Stators gebrochen werden, oder durch den Winkelabstand der Rotorpole. Durch diese Massnahme sollen solche relativen Drehpositionen zwischen Rotor und Stator vermieden werden, bei denen es durch die Symmetrie bedingt nicht mehr möglich ist, mit dem Stator ein resultierendes Drehmoment auf den Rotor auszuüben, bei denen der Rotor also beim Stillstand einrastet.

Eine weitere vorteilhafte Massnahme besteht darin, dass der magnetisch wirksame Kern des Rotors ringförmig ausgestaltet ist, wobei in der Mitte bezüglich der axialen Richtung ein umlaufender Ring konstanten Durchmessers ausgebildet ist und wobei die Rotorpole oberhalb und unterhalb des Rings vorgesehen sind. Mit dieser Massnahme kann die Anzahl der Positionssensoren im Stator vermindert werden, mit welchen die radiale Position des Rotors relativ zum Stator bestimmt wird.

Für die Erzeugung der elektromagnetischen Felder bzw. der Drehfelder ist es bevorzugt, dass auf jedem oberen und auf jedem unteren Statorpol jeweils eine Spule als Wicklung angeordnet ist, wobei für jede Spule jeweils ein separater Leistungsverstärker vorgesehen ist, mit welchem der Spulenstrom oder die Spulenspannung für die Spule jeweils unabhängig von den Spulenströmen oder den Spulenspannungen der anderen Spulen regelbar ist. Diese Massnahme ist besonders vorteilhaft, um mit dem Stator sowohl ein Drehmoment auf den Rotor auszuüben, als auch eine beliebig einstellbare Querkraft in radialer Richtung, mit welcher die radiale Position des Rotors - also sein Lage in der Ebene senkrecht zur Solldrehachse - aktiv magnetisch regelbar ist. Die unabhängige Ansteuerbarkeit jeder Spule ermöglicht es zudem, dass durch das Zusammenwirken des oberen und des unteren Statorteils die Lage des Rotors bezüglich Verkippungen gegen die Solldrehachse (zwei Freiheitsgrade) aktiv magnetisch regelbar ist.

Bezüglich der Ausgestaltung des Stators ist es ganz besonders bevorzugt, wenn der Permanentmagnet jedes Stators scheibenförmig oder ringförmig ausgestaltet ist, in axialer Richtung magnetisiert ist, und jeweils den oberen Statorteil mit dem unteren Statorteil verbindet. D permanentmagnetisch erzeugte Fluss selbstverständlich auch durch zusätzliche weichmagnetische Teile geführt werden kann. Der Permanentmagnet kann ebenfalls aus mehreren Einzelmagneten wie beispielsweise Segmentmagneten oder Blockmagneten aufgebaut sein.

Bevorzugte Anwendungen sind es, wenn der Drehantrieb als Pump- oder Mischvorrichtung zum Fördern oder Mischen von fluiden Substanzen, oder als Bestandteil einer Pump-oder Mischvorrichtung zum Fördern oder Mischen von fluiden Substanzen ausgestaltet ist.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der Zeichnung zeigen (teilweise im Schnitt):
- Fig. 1:: eine perspektivische Schnittdarstellung eines ersten Ausführungsbeispiels eines erfindungsgemässen elektromagnetischen Drehantriebs.
- Fig. 2:: eine Aufsicht auf den Stator und den magnetisch wirksamen Kern des Rotors des ersten Ausführungsbeispiels aus Richtung der Solldrehachse,
- Fig. 3: eine schematische Darstellung eines Ausführungsbeispiels eines Leistungsverstärkers zum Regeln des Spulenstroms oder der Spulenspannung,
- Fig. 4: eine schematische Darstellung einer Variante für die Leistungsverstärker zum Regeln der sechs Spulenströme oder Spulenspannungen des ersten Ausführungsbeispiels,
- Fig. 5:: eine perspektivische Schnittdarstellung einer ersten Variante des ersten Ausführungsbeispiels mit einer alternativen Ausgestaltung des magnetisch wirksamen Kerns des Rotors,
- Fig. 6:: eine Aufsicht auf den Stator und den magnetisch wirksamen Kern des Rotors aus Richtung der Solldrehachse für eine zweite Variante des ersten Ausführungsbeispiels mit einer alternativen Ausgestaltung des magnetisch wirksamen Kerns des Rotors,
- Fig. 7:: eine Aufsicht auf den Stator und den magnetisch wirksamen Kern des Rotors aus Richtung der Solldrehachse für eine dritte Variante des ersten Ausführungsbeispiels mit einer alternativen Ausgestaltung des magnetisch wirksamen Kerns des Rotors,
- Fig. 8:: eine Aufsicht auf den Stator und den magnetisch wirksamen Kern des Rotors aus Richtung der Solldrehachse für eine vierte Variante des ersten Ausführungsbeispiels mit einer alternativen Ausgestaltung des magnetisch wirksamen Kerns des Rotors,
- Fig. 9:: eine Aufsicht auf den Stator und den magnetisch wirksamen Kern des Rotors aus Richtung der Solldrehachse für eine erste Variante des ersten Ausführungsbeispiels mit einer alternativen Ausgestaltung des Stators,
- Fig. 10:: eine perspektivische Ansicht einer zweiten Variante für die Ausgestaltung des Stators des ersten Ausführungsbeispiels,
- Fig. 11:: eine Aufsicht auf den Stator aus Fig. 10 zusammen mit dem magnetisch wirksamen Kern des Rotors,
- Fig. 12:: wie Fig. 4, jedoch für den Stator gemäss Fig. 10,
- Fig. 13:: eine schematische Darstellung für eine Variante der Leistungsverstärker zum Regeln der Spulenströme oder Spulenspannungen des Stators gemäss Fig. 10,
- Fig. 14:: eine schematische Darstellung für eine weitere Variante der Leistungsverstärker zum Regeln der Spulenströme oder Spulenspannungen des Stators gemäss Fig. 10,
- Fig. 15:: eine perspektivische Ansicht einer dritten Variante für die Ausgestaltung des Stators des ersten Ausführungsbeispiels,
- Fig. 16:: eine Aufsicht auf den Stator aus Fig. 15 zusammen mit dem magnetisch wirksamen Kern des Rotors
- Fig. 17:: wie Fig. 12, jedoch für den Stator gemäss Fig. 16,
- Fig. 18:: wie Fig. 14, jedoch für den Stator gemäss Fig. 16,
- Fig. 19:: eine perspektivische Ansicht einer vierten Variante für die Ausgestaltung des Stators des ersten Ausführungsbeispiels,
- Fig. 20:: eine Aufsicht auf den Stator aus Fig. 19 zusammen mit dem magnetisch wirksamen Kern des Rotors,
- Fig. 21:: wie Fig. 18, jedoch für den Stator gemäss Fig. 20,
- Fig. 22:: eine perspektivische Schnittdarstellung eines zweiten Ausführungsbeispiels des erfindungsgemässen Drehantriebs ausgestaltet als Pump- oder Mischvorrichtung,
- Fig. 23:: eine perspektivische Schnittdarstellung eines dritten Ausführungsbeispiels des erfindungsgemässen Drehantriebs ausgestaltet als Pump- oder Mischvorrichtung,
- Fig. 24:: eine perspektivische Schnittdarstellung eines vierten Ausführungsbeispiels des erfindungsgemässen Drehantriebs integriert in eine Mischvorrichtung,

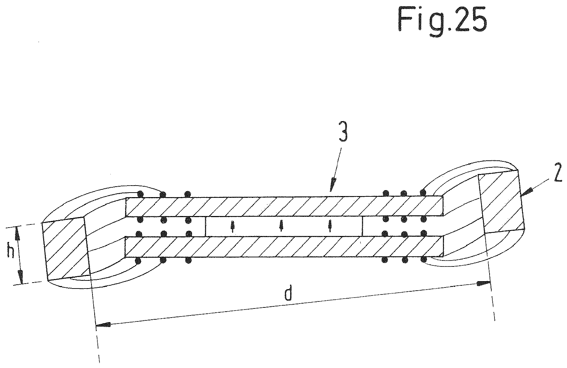

- Fig. 25:: eine schematische Darstellung zur Veranschaulichung der passiven magnetischen Stabilisierung des Rotors gegen Verkippungen, und

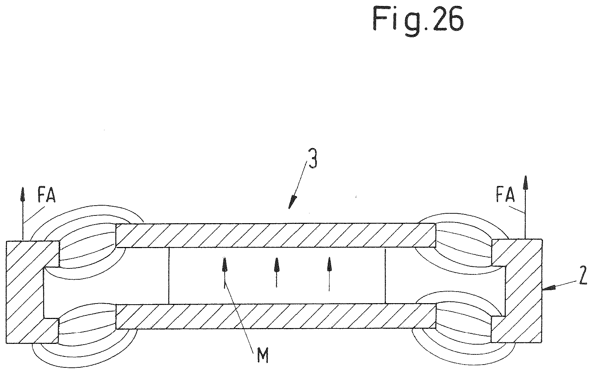

- Fig. 26:: eine schematische Darstellung zur Veranschaulichung der passiven magnetischen Stabilisierung der axialen Position des Rotors.

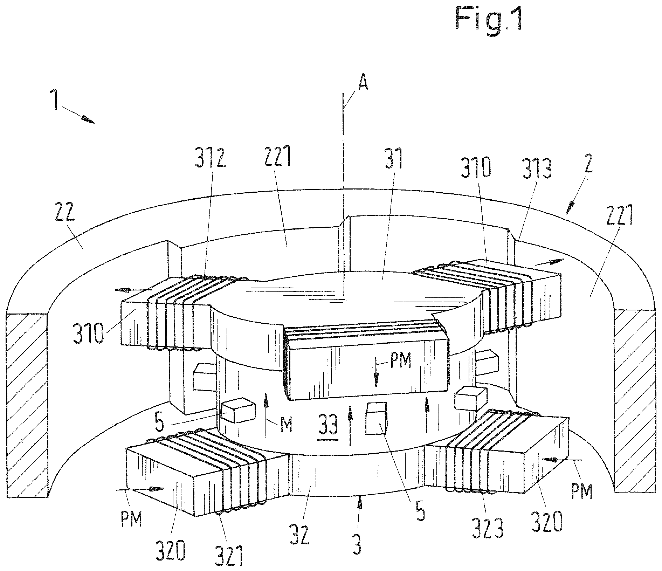

Fig. 1 zeigt in einer perspektivischen Schnittdarstellung ein erstes Ausführungsbeispiel eines erfindungsgemässen elektromagnetischen Drehantriebs, der gesamthaft mit dem Bezugszeichen 1 bezeichnet ist. Der Drehantrieb 1 ist nach dem Prinzip des lagerlosen Motors ausgestaltet und umfasst einen Rotor 2, welcher berührungslos magnetisch antreibbar ist und spulenfrei ausgestaltet ist, sowie einen Stator 3, welcher als Lager- und Antriebsstator ausgestaltet ist, mit welchem der Rotor 2 im Betriebszustand berührungslos magnetisch um eine Solldrehachse A antreibbar und bezüglich des Stators 3 berührungslos magnetisch lagerbar ist. Bei dem hier beschriebenen Ausführungsbeispiel ist der Stator 3 innenliegend bezüglich des Rotors 2 angeordnet.

Im Folgenden wird mit der Solldrehachse A diejenige Drehachse bezeichnet, um welche der Rotor 2 rotiert, wenn er sich bezüglich des Stators 3 in einer zentrierten Position befindet. Der Rotor 2 ist dann in einer Ebene zentriert, welche senkrecht zur Mittelachse des Stators 3 ist, und bezüglich dieser Ebene nicht verkippt. Die Solldrehachse A fällt in der Regel mit der Mittelachse des Stators 3 zusammen.

Ferner wird im Folgenden die durch die Solldrehachse A definierte Richtung als axiale Richtung bezeichnet, die dazu senkrechten Richtungen werden allgemein mit radialer Richtung bezeichnet. Mit der radialen Ebene wird diejenige zur Solldrehachse A senkrechte Ebene bezeichnet, welche die magnetische Mittelebene des Stators 3 ist. Die radiale Ebene definiert die x-y -Ebene eines kartesischen Koordinatensystems, dessen z-Achse in axialer Richtung verläuft.

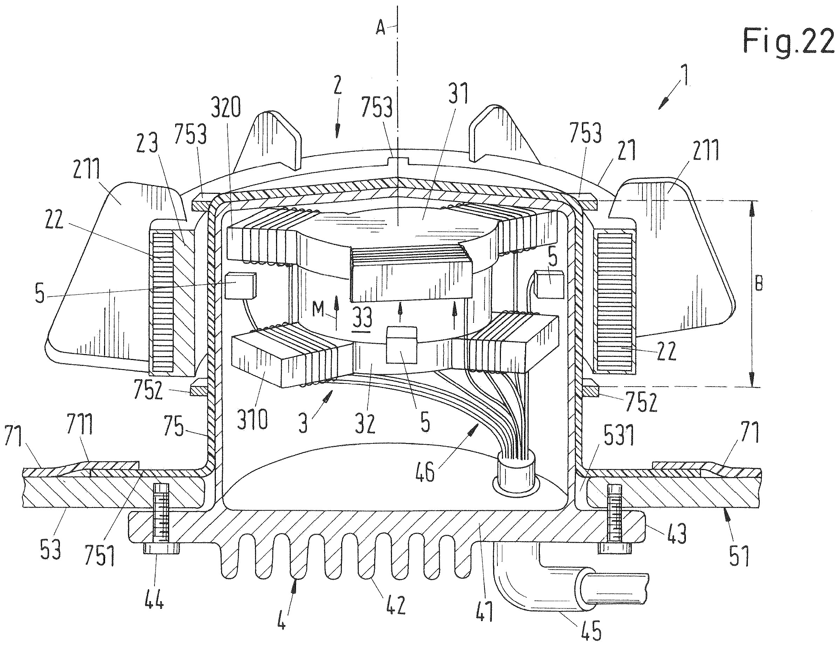

Der Rotor 2 des erfindungsgemässen Drehantriebs 1 ist spulenfrei ausgestaltet, d. h. auf dem Rotor 2 sind keine Wicklungen vorgesehen. Der Rotor 2 umfasst einen magnetisch wirksamen Kern 22, der je nach Ausgestaltung von einer Kunststoffummantelung umgeben sein kann. Beispiele für die Ausgestaltung des Rotors werden weiter hinten noch im Zusammenhang mit den Fig. 22-24 erläutert. Da es für das Verständnis der Erfindung ausreichend ist, wird in den meisten Zeichnungsfiguren nur der magnetisch wirksame Kern 22 des Rotors dargestellt. Es versteht sich aber, dass der Rotor 2 natürlich noch andere Komponenten wie beispielsweise eine bereits erwähnte Ummantelung umfassen kann.

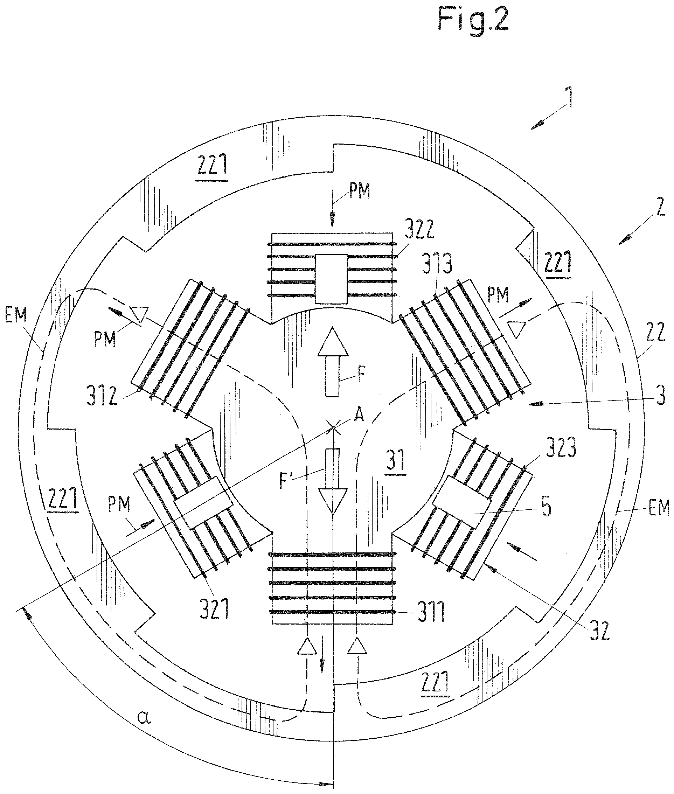

Zum besseren Verständnis zeigt Fig. 2 noch eine Aufsicht aus der axialen Richtung - also aus der Richtung der Solldrehachse A - auf den Stator 3 und den magnetisch wirksamen Kern 22 des Rotors 2.

In einer ganz besonders bevorzugten Ausgestaltung der Erfindung, die auch in dem hier beschriebenen Ausführungsbeispiel realisiert ist, weist der Rotor 2 bzw. der magnetisch wirksame Kern 22 des Rotors 2 keine Permanentmagnete auf, er ist also frei von Permanentmagneten. Diese Massnahme ermöglicht eine besonders kostengünstige Ausgestaltung des Rotors 2 - beispielsweise auch als Einmalteil -, denn insbesondere sind für die Herstellung des Rotors 2 keine Seltenen Erden wie z. B. Neodym oder Samarium, bzw. Verbindungen oder Legierungen dieser notwendig, die häufig für die Herstellung von Permanentmagneten verwendet werden. Der Verzicht auf diese Permanentmagnete im Rotor bedeutet auch unter Umweltaspekten einen grossen Vorteil.

Als Permanentmagnete bezeichnet man üblicherweise solche ferromagnetischen oder ferrimagnetischen Werkstoffe, die hartmagnetisch sind, also eine hohe Koerzitivfeldstärke aufweisen. Die Koerzitivfeldstärke ist diejenige magnetische Feldstärke, die man benötigt, um einen Stoff zu entmagnetisieren. Im Rahmen dieser Anmeldung wird unter einem Permanentmagneten ein Werkstoff verstanden, der eine Koerzitivfeldstärke, genauer gesagt eine Koerzitivfeldstärke der magnetischen Polarisation aufweist, die mehr als 10'000 A/m beträgt.

Wenn der Rotor 2 also frei von Permanentmagneten ist, so bedeutet dies, dass der magnetisch wirksame Kern 22 des Rotors nur Materialien umfasst, deren Koerzitivfeldstärke höchstens 10'000 A/m beträgt.

Mit der Bezeichnung, dass der Rotor 2 "frei von Permanentmagneten" ist, soll im Rahmen dieser Anmeldung verstanden werden, dass der Rotor 2 keine Permanentmagnete umfasst, die einen wesentlichen Beitrag zum Antriebsfeld zum Antreiben der Rotation des Rotors 2 leisten. Es ist natürlich möglich, dass am Rotor 2 andere Magnete bzw. Permanentmagnete vorgesehen sind, welche beispielsweise nur der Erfassung der Winkelstellung des Rotors dienen oder die sonst einen Zweck erfüllen, der nichts mit dem Generieren des Antriebsflusses für den Rotors zu tun hat. Die Bezeichnung "frei von Permanentmagneten" bezieht sich also nur auf den Antrieb des Rotors 2.

Vorzugsweise ist der magnetisch wirksame Kern 22 des Rotors aus einem weichmagnetischen Material gefertigt, beispielsweise aus Eisen, Nickel-Eisen oder Silizium-Eisen. Dabei kann der magnetisch wirksame Kern 22 z.B. durch Giessen, Stanzen, Pressen von weichmagnetischem Pulver mit anschliessendem Sintern, Schmieden, Umformen oder Zusammensetzen von Teilen wie Blechen hergestellt werden.

Bei dem ersten Ausführungsbeispiel ist der magnetisch wirksame Kern 22 im wesentlichen ringförmig ausgestaltet (siehe auch Fig. 2), wobei über den inneren Umfang des ringförmigen Kerns 22 verteilt mehrere ausgeprägte Rotorpole 221 vorgesehen sind, die sich radial nach innen erstrecken. Bezüglich der axialen Richtung erstreckt sich jeder Rotorpol 221 über die gesamte axiale Höhe des ringförmigen magnetisch wirksamen Kerns 22. Bei der hier beschriebenen Variante hat der magnetisch wirksame Kern 22 vier Rotorpole 221, die äquidistant über den inneren Umfang des ringförmigen magnetisch wirksamen Kerns verteilt sind.

Der Stator 3 ist als Lager- und Antriebsstator ausgestaltet, mit welchem der Rotor 2 im Betriebszustand berührungslos magnetisch um die Solldrehachse A antreibar -also in Rotation versetzbar- ist und bezüglich des Stators 3 berührungslos magnetisch lagerbar ist. Der Stator 3 und der Rotor 2 bilden somit einen elektromagnetischen Drehantrieb, der gleichzeitig eine magnetische Lagerung des Rotors 2 ermöglicht. Dieser elektromagnetische Drehantrieb 1 ist besonders bevorzugt nach dem Prinzip des lagerlosen Motors ausgestaltet. Der lagerlose Motor ist dem Fachmann mittlerweile hinlänglich bekannt, sodass eine detaillierte Beschreibung seiner Funktion nicht mehr notwendig ist.

Mit dem Begriff lagerloser Motor ist gemeint, dass der Rotor 2 vollkommen magnetisch gelagert ist, wobei keine separaten Magnetlager vorgesehen sind. Der Stator 3 ist dazu als Lager- und Antriebsstator ausgestaltet, er ist also sowohl Stator des elektrischen Antriebs als auch Stator der magnetischen Lagerung. Dabei umfasst der Stator 3 Wicklungen, mit denen sich ein magnetisches Drehfeld erzeugen lässt, welches zum einen ein Drehmoment auf den Rotor 2 ausübt, das dessen Rotation bewirkt, und welches zum anderen eine beliebig einstellbare Querkraft auf den Rotor 2 ausübt, sodass dessen radiale Position -also seine Position in der radialen Ebene- aktiv steuerbar bzw. regelbar ist. Somit sind zumindest drei Freiheitsgrade des Rotors 2 aktiv regelbar. Bezüglich seiner axialen Auslenkung in Richtung der Solldrehachse A ist der Rotor 2 passiv magnetisch, das heisst nicht ansteuerbar, durch Reluktanzkräfte stabilisiert. Auch bezüglich der verbleibenden zwei Freiheitsgrade, nämlich Verkippungen bezüglich der zur Solldrehachse A senkrechten radialen Ebene kann der Rotor 2 -je nach Ausführungsform - ebenfalls passiv magnetisch stabilisiert sein.

Aus dem Stand der Technik, beispielsweise aus der US-A-2009/121571 ist eine elektromagnetische Antriebs- und Lagervorrichtung bekannt, bei welcher der Stator des Antriebs und der Stator der magnetischen Lagerung zu einer baulichen Einheit zusammengefügt sind. Hier umfasst der Stator eine Lagereinheit, die aus einer oberen und einer unteren Lagerebene besteht, sowie eine Antriebseinheit, die zwischen diesen Lagerebenen angeordnet sind. Auch diese Vorrichtung zeigt also eine von der Antriebseinheit separierbare Lagereinheit, die ausschliesslich der magnetischen Lagerung dient. Derartige Vorrichtungen sind jedoch nicht als lagerlose Motoren im Sinne der vorliegenden Anmeldung zu verstehen, weil hier tatsächlich separate Lagereinheiten vorhanden sind, welche getrennt von der Antriebsfunktion die Lagerung des Rotors realisieren. Bei einem lagerlosen Motor im Sinne der vorliegenden Anmeldung ist es nicht möglich, den Stator in eine Lagereinheit und in eine Antriebseinheit aufzuteilen. Gerade diese Eigenschaft ist es ja, die dem lagerlosen Motor seinen Namen gibt.

Beim lagerlosen Motor wird im Unterschied zu klassischen Magnetlagern die magnetische Lagerung und der Antrieb des Motors über elektromagnetische Drehfelder realisiert, deren Summe zum einen ein Antriebsmoment auf den Rotor 2 erzeugen, sowie eine beliebig einstellbare Querkraft, mit welcher die radiale Position des Rotors 2 regelbar ist. Diese Drehfelder können entweder separat - also mit unterschiedlichen Spulen -, generiert werden, wobei die Lagerung nur durch die Kombination der von den beiden generierten Drehfelder realisierbar ist, oder die Drehfelder können durch rechnerische Überlagerung der benötigten Ströme und dann mithilfe eines einzigen Spulensystems generiert werden. Es ist also nicht möglich, den elektromagnetischen Fluss, der von den Spulen des Stators generiert wird, aufzuteilen in einen elektromagnetischen Fluss, der nur für den Antrieb des Rotors sorgt und einen elektromagnetischen Fluss, der nur die magnetische Lagerung des Rotors realisiert.

Der lagerlose Motor kann als Innenläufer, das heisst mit innenliegendem Rotor und darum herum angeordneten Stator, oder als Aussenläufer, das heisst mit innenliegendem Stator 2 und darum herum angeordneten Rotor 3 ausgestaltet sein. Bei dem hier beschriebenen ersten Ausführungsbeispiel ist der elektromagnetische Drehantrieb 1 als Aussenläufer ausgestaltet.

Erfindungsgemäss umfasst der Stator 3 einen oberen Statorteil 31 mit einer Mehrzahl von ausgeprägten oberen Polen 310 zum Tragen von oberen Wicklungen 311, 312, 313 sowie einen unteren Statorteil 32 mit einer Mehrzahl von ausgeprägten unteren Polen 320 zum Tragen von unteren Wicklungen 321, 322, 323. Der obere Statorteil 31 und der untere Statorteil 32 sind bezüglich der axialen Richtung beabstandet zueinander angeordnet. Zwischen dem oberen Statorteil 31 und dem unteren Statorteil 32 ist ein Permanentmagnet 33 vorgesehen.

Ein wesentlicher Aspekt, der dem Prinzip des lagerlosen Motors Rechnung trägt, ist es dabei, dass sowohl der obere Statorteil 31 als auch der untere Statorteil 32 jeweils zum Antrieb und zur berührungslosen magnetischen Lagerung des Rotors beitragen.

Vorzugsweise sind der axiale Abstand des oberen 31 vom unteren Statorteil 32 und die Dicke des oberen und des unteren Statorteils 31, 32 so bemessen, dass die gesamte axiale Höhe des Stators 3 zumindest ungefähr gleich gross, und vorzugsweise gleich gross ist, wie die Höhe des magnetisch wirksamen Kerns 22 des Rotors 2 in axialer Richtung.

Bei dem hier beschriebenen und in den Fig. 1 und 2 dargestellten ersten Ausführungsbeispiel umfasst der Stator 3 genau drei obere Pole 310 und genau drei untere Pole 320. Der obere und der untere Statorteil 31, 32 sind im Wesentlichen identisch ausgebildet und jeweils im wesentlichen scheibenförmig ausgestaltet, wobei sich jeder Pol 310, 320 von dem zentralen Scheibenteil in radialer Richtung nach aussen erstreckt. Der obere und der untere Statorteil 31, 32 sind jeweils aus einem weichmagnetischen Material, beispielsweise Eisen, gefertigt und können auch als Statorblechpaket ausgestaltet sein. Der obere und der untere Statorteil 31, 32 liegen parallel zueinander, wobei zwischen ihnen der Permanentmagnet 33 angeordnet ist, der scheibenförmig oder ringförmig ausgestaltet ist und den unteren Statorteil 32 mit dem oberen Statorteil 31 verbindet. Der Permanentmagnet 33 ist in axialer Richtung - darstellungsgemäss von unten nach oben - magnetisiert, wie dies die Pfeile mit den Bezugszeichen M andeuten. Der äussere Durchmesser des Permanentmagneten 33 ist so bemessen, dass er vorzugsweise nicht grösser ist, und besonders bevorzugt etwas kleiner ist, als der Durchmesser des zentralen Scheibenteils des oberen bzw. unteren Statorteils 31, 32. Mit dem zentralen Scheibenteil ist derjenige Teil des oberen bzw. unteren Statorteils 31, 32 gemeint, der übrig bleibt, wenn man sich die Pole 310 bzw. 320 weg denkt.

Die im Wesentlichen identischen oberen und unteren Statorteile 31 und 32 sind bei der in den Fig. 1 und Fig. 2 dargestellten Variante des ersten Ausführungsbeispiels bezüglich der Solldrehachse A relativ zueinander um eine Winkel α verdreht angeordnet (siehe Fig. 2), sodass die oberen Pole 310 in axialer Richtung gesehen jeweils in einer Lücke zwischen zwei benachbarten unteren Polen 320 angeordnet sind. Bei der in den Fig. 1 und 2 dargestellten Ausführungsform beträgt der Winkel α 60°, sodass jeder obere Pol 310 in axialer Richtung betrachtet genau mittig zwischen jeweils zwei benachbarten Polen 320 des unteren Statorteils 32 angeordnet ist. Eine solche besonders symmetrische Ausgestaltung lässt sich auch für andere Anzahlen von Statorpolen 310, 320 erzielen. Wenn N die Gesamtzahl aller oberen und unteren Pole 310, 320 bezeichnet, dann wird der Winkel α für diese symmetrische Anordnung nach der Beziehung α = 360°/N bestimmt.

Diese verdrehte Anordnung des oberen 31 relativ zum unteren Statorteils 32 hat insbesondere den Vorteil, dass bei einer kleineren Anzahl von oberen und unteren Polen 310, 320, beispielsweise bei je drei oder je vier unteren und oberen Polen 310, 320 durch das Zusammenspiel der beiden Statorteile 31, 32 für jede relative Winkelposition des Rotors 2 zum Stator 3 mit einem der beiden Statorteile 31 oder 32 eine Krafteinwirkung auf den Rotor 2 generiert werden kann, während mit dem anderen der beiden Statorteile 32 oder 31 ein Drehmoment auf den Rotor 2 generiert werden kann, wie weiter hinten noch erläutert wird.

Auf jedem der unteren und der oberen Pole 320, 310 ist jeweils eine Spule 321, 322, 323, 311, 312, 313 als Wicklung vorgesehen, mit denen ein elektromagnetisches Drehfeld erzeugbar ist, das ein Drehmoment auf den Rotor 2 ausübt. Gleichzeitig ist mit den Spulen 321, 322, 323, 311, 312, 313 gemäss dem Prinzip des lagerlosen Motors eine beliebig einstellbare Querkraft auf den Rotor 2 ausübbar, mit welcher die Position des Rotors 2 in der radialen Ebene aktiv magnetisch regelbar ist.

Ferner sind Positionssensoren 5 vorgesehen, mit welchen die radiale Position des Rotors 2 - also seine Lage in der radialen oder der x-y-Ebene - ermittelbar ist. Die Positionssensoren 5 sind vorzugsweise als Hallsensoren oder Wirbelstromsensoren ausgestaltet und über nicht näher bezeichnete Signalleitungen mit einer nicht dargestellten Kontroll- und Regeleinrichtung signalverbunden.

Es ist eine übliche und bekannte Massnahme, insgesamt vier Positionssensoren 5 vorzusehen, um die Position des Rotors 2 zu bestimmen. Dabei liegen sich die Positionssensoren 5 paarweise diametral gegenüber. Prinzipiell sind zwei Positionssensoren 5 ausreichend, um die Position des Rotors 2 in der x-y-Ebene zur ermitteln, nämlich einer pro Koordinatenrichtung. Es ist jedoch bevorzugt, vier Positionssensoren 5 vorzusehen, um so aus dem Differenzsignal der sich paarweise gegenüberliegenden Positionssensoren 5 eine genauere Bestimmung der Lage des Rotors 2 zu ermöglichen.

Eine solche Anordnung des Positionssensoren 5 ist natürlich auch bei dem erfindungsgemässen Drehantrieb möglich. In Fig. 1 ist ein Variante gezeigt, bei der eine andere Anordnung der Positionssensoren 5 vorgesehen ist. Bei dieser Variante sind die Positionssensoren 5 im Stator 3 angeordnet, etwa in der Mitte zwischen dem oberen und dem unteren Statorteil 31, 32, und vorzugsweise äquidistant über den äusseren Umfang des Permanentmagneten 33 verteilt. Bei dieser Anordnung sind mindestens fünf Positionssensoren 5 notwendig, wobei eine Ausgestaltung mit sechs oder acht Positionssensoren 5 bevorzugt ist. Mit dieser Anordnung der Positionssensoren 5 kann mit Hilfe der Sensorsignale sowohl die radiale Position des Rotors 2 als auch der Drehwinkel des Rotors 2 bestimmt werden. Bei den Positionssensoren 5 kann es sich beispielsweise jeweils um Wirbelstromsensoren, optische Sensoren, kapazitive Sensoren oder Magnetfeldsensoren wie Hallsensoren oder GRM Sensoren handeln. Bei Magnetfeldsensoren wird vorzugsweise hinter dem Sensor ein kleiner Permanentmagnet angeordnet, falls das Streufeld des Permanentmagneten 33 nicht ausreichen sollte.

Zum besseren Verständnis ist in den Fig. 1 und 2 der Verlauf des von dem Permanentmagneten 33 generierten permanentmagnetischen Flusses durch die mit dem Bezugszeichen PM versehenen Pfeile schematisch angedeutet. Der permanentmagnetische Fluss verläuft in axialer Richtung darstellungsgemäss (Fig. 1) nach oben durch den Permanentmagneten 33, wird dann im oberen Statorteil 31 radial nach aussen durch die oberen Pole 310 in den magnetisch wirksamen Kern 22 des Rotors 2 geführt, verläuft dort in axialer Richtung darstellungsgemäss nach unten und wird am unteren axialen Ende des magnetisch wirksamen Kerns 22 radial nach innen in die unteren Pole 320 des unteren Statorteils 32 geführt, von wo er in die axiale Richtung zurück in den Permanentmagneten geführt wird.

In Fig. 2 ist mit beispielhaftem Charakter für eine Drehstellung des Rotors 2 relativ zum Stator 3 die Erzeugung einer radial nach aussen wirkenden Querkraft F' auf den Rotor 2 veranschaulicht. Mit F ist die entgegengesetzt gleich grosse Kraft bezeichnet, die auf den Stator 3 wirkt. Hierzu ist neben dem permanentmagnetischen Fluss, der mit den Pfeilen mit dem Bezugszeichen PM angedeutet ist, auch der mit den auf den oberen Polen 310 angeordneten Spulen 311, 312, 313 generierte elektromagnetische Fluss durch die strichliert dargestellten Linien mit den Bezugszeichen EM angedeutet.

Wie dies zu erkennen ist, erzeugt der permanentmagnetische Fluss PM aufgrund der symmetrischen Krafteintragung keine resultierende Kraft auf den Rotor 2. Der mit den Spulen 311, 312, 313 generierte elektromagnetische Fluss EM tritt in dieser Momentaufnahme an den Spulen 312 und 313 radial nach aussen aus dem Stator 3 aus und in die jeweils gegenüberliegenden Rotorpole 221 in den Rotor 2 ein. An der Spule 311 tritt dann der gesamte elektromagnetische Fluss EM aus dem Rotorpol 221 aus und wird radial nach innen in den oberen Pol 310, der die Spule 311 trägt, geführt. In der Summe resultiert dadurch die radial nach aussen wirkende Querkraft F auf den Stator 3, die in Fig. 2 durch den Pfeil mit dem Bezugszeichen F angedeutet ist und eine in der entgegengesetzten Richtung wirkende Querkraft auf den Rotor 2 . Anhand dieses Beispiels ist zu erkennen, wie mit dem Stator 3 eine beliebig einstellbare Querkraft auf den Rotor 2 generierbar ist, mit welcher die radiale Position des Rotors 2 - also seine Position in der radialen Ebene - aktiv magnetisch regelbar ist. Es ist ebenfalls ersichtlich, dass der elektromagnetische Fluss EM ausser im Bereich der Luftspalte zwischen Rotor 2 und Stator 3 immer durch weichmagnetisches Material geführt wird und nicht durch den Permanentmagneten 33 führt.

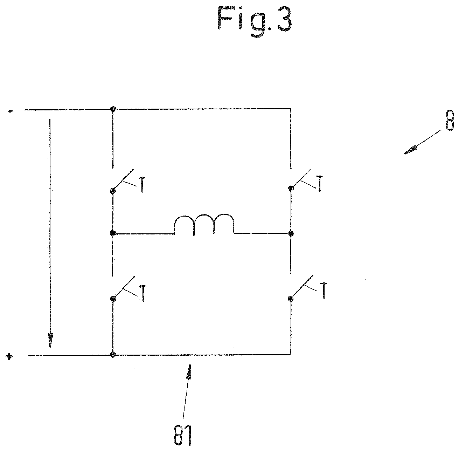

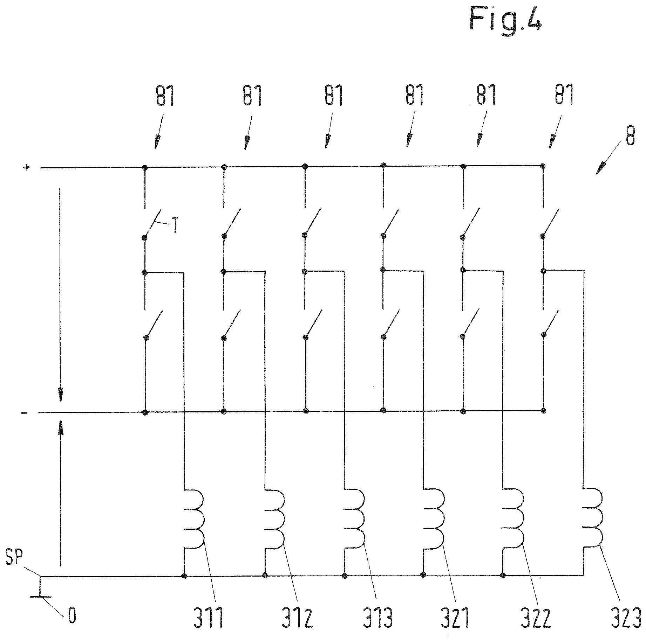

Für die Ansteuerung der Spulen 311, 312, 313, 321, 322, 323 zum Generieren des elektromagnetischen Drehfelds ist eine nicht dargestellte Stelleinrichtung vorhanden, die eine Verstärkereinheit 8 (siehe Fig. 3, Fig. 4) umfasst und von der (nicht dargestellten) Kontroll- und Regeleinrichtung angesteuert wird. Für die Ausgestaltung der Verstärkereinheit 8 gibt es mehrere Varianten, von denen im Folgenden zwei beschrieben werden, die für das erste Ausführungsbeispiel mit den insgesamt sechs Spulen 311, 312, 313, 321, 322, 323 geeignet sind. Dabei ist es vorteilhaft, wenn für jede der Spulen 311, 312, 313, 321, 322, 323 jeweils ein separater Leistungsverstärker 81 vorgesehen ist, mit welchem der Spulenstrom oder die Spulenspannung für diese Spule unabhängig von den Spulenströmen oder den Spulenspannungen der anderen Spulen regelbar ist.

Im Folgenden wird mit beispielhaftem Charakter auf den Fall Bezug genommen, dass jeweils der Spulenstrom als Stellgrösse geregelt wird.

Bei der hier beschriebenen Ausgestaltung wird auf den Fall Bezug genommen, dass jede der Spulen 311, 312, 313, 321, 322, 323 jeweils als genau eine diskrete Spule ausgestaltet, die für sich genommen eine elektrische Phase bildet. Es sind natürlich auch solche Ausgestaltungen möglich, bei denen einige oder jede der Spulen 311, 312, 313, 321, 322, 323 jeweils mehr als eine diskrete Spule umfassen.

Da bei der hier beschriebenen Ausgestaltung also sechs Spulen 311, 312, 313, 321, 322, 323 vorgesehen sind, die jeweils zu einer separaten elektrischen Phase gehören, müssen in der Verstärkereinheit 8 insgesamt sechs Leistungsverstärker 81 vorgesehen sein.

Bei der in Fig. 3 dargestellten Variante umfasst die Verstärkereinheit 8 insgesamt sechs Leistungsverstärker 81, nämlich für jede elektrische Phase einen. Jeder Leistungsverstärker 81 ist ein bipolarer Leistungsverstärker 81, der jeweils in an sich bekannter Weise als H-Brückenschaltung ausgestaltet ist. In Fig. 3 ist nur eine dieser H-Brückenschaltungen dargestellt, weil die Schaltbilder der anderen fünf identisch aussehen.

Mit der Bezeichnung "bipolarer Leistungsverstärker" ist gemeint, dass sowohl die Phasenströme als auch die Phasenspannungen jeweils positives und negatives Vorzeichen annehmen können.

Die H-Brückenschaltungen (siehe Fig. 3) sind in an sich bekannter Weise mit Schalttransistoren T und nicht dargestellten Freilaufdioden realisiert und werden mit den Betriebspotentialen + und - betrieben. Das Betriebspotential - ist beispielsweise das Erdpotential.

Fig. 4 zeigt eine andere Variante für die Leistungsverstärker 81 der Verstärkereinheit 8 zum separaten Regeln der Spulenströme (oder Spulenspannungen) in den Spulen 311, 312, 313, 321, 322, 323. Bei dieser Variante ist jeder der sechs Leistungsverstärker 81 jeweils ein Brückenzweig der Verstärkereinheit 8. Für jede der Spulen 311, 312, 313, 321, 322, 323 bzw. für jede der separaten elektrischen Phasen ist jeweils ein Brückenzweig der Verstärkereinheit 8 als separater bipolarer Leistungsverstärker 81 vorgesehen. Jeder Brückenzweig kann in an sich bekannter Weise mittels Schalttransistoren T und (nicht dargestellten) Freilaufdioden eine der Spulen 311, 312, 313, 321, 322, 323 mit dem jeweiligen Spulenstrom oder der jeweiligen Spulenspannung versorgen. Die Verstärkereinheit 8 wird mit zwei Betriebspotentialen betrieben, die in Fig. 4 mit + und - bezeichnet sind. Diese Betriebspotentiale +,- sind Gleichspannungspotentiale. Zwischen diesen beiden Betriebspotentialen liegt das Mittelpunktpotential O, das beispielsweise das Erdpotential ist. Jede Spule 311, 312, 313, 321, 322, 323 ist einerseits mit dem sie versorgenden bipolaren Leistungsverstärker 81 verbunden. Andererseits ist jede Spule 311, 312, 313, 321, 322, 323 mit einem gemeinsamen Sternpunkt SP verbunden, der auf dem Mittelpunktpotential O liegt. Vorzugsweise, aber nicht notwendigerweise, ist der Sternpunkt SP als belastbarer Sternpunkt SP ausgestaltet, das heisst er ist mit einem belastbaren Potential verbunden, sodass, abgesehen von den sechs Spulenströmen ein zusätzlicher Strom über den Sternpunkt SP abfliessen bzw. in diesen hineinfliessen kann. Das bedeutet, die übliche Sternpunktbedingung, dass die Summe der Spulenströme im Sternpunkt SP immer Null sein muss, ist bei dieser Schaltung nicht mehr notwendig. Dies hat zur Folge, dass jeder Spulenstrom vollkommen unabhängig von den anderen Spulenströmen geregelt werden kann.

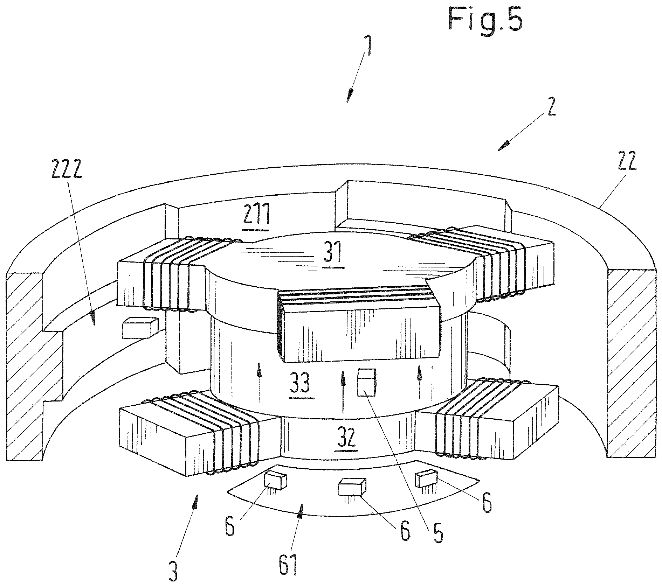

Fig. 5 zeigt in einer perspektivischen Schnittdarstellung des Stators 3 und des magnetisch wirksamen Kerns 22 des Rotors 2 eine erste Variante des ersten Ausführungsbeispiels mit einer alternativen Ausgestaltung des magnetisch wirksamen Kerns 22 des Rotors 2. Auch bei dieser Variante des magnetisch wirksamen Kerns 22 des Rotors 2 ist dieser im Wesentlichen ringförmig ausgestaltet, wobei jedoch in der Mitte (bezogen auf die axiale Richtung) ein umlaufender Ring 222 vorgesehen ist, der einen über den gesamten Umfang konstanten Innendurchmesser aufweist. Oberhalb und unterhalb des Rings 222 sind dann wie bereits beschrieben die Rotorpole 221 - hier vier- vorgesehen.

Diese Ausgestaltung ermöglicht es, die Anzahl der Positionssensoren 5 im Vergleich zu der in Fig. 1 dargestellten Variante zu reduzieren. Prinzipiell ist es möglich bei der Ausgestaltung mit dem zylindrischen, umlaufenden Ring 222 mit nur zwei Positionssensoren 5 auszukommen, nämlich einen für jede der beiden kartesischen Koordinatenachsen der radialen Ebene. Um die Genauigkeit der Positionsbestimmung zu erhöhen, ist es aber auch hier bevorzugt, insgesamt vier Positionssensoren 5 vorzusehen, von denen je zwei paarweise diametral gegenüber liegend angeordnet sind, und dann jeweils das Differenzsignal dieser beiden gegenüberliegenden Positionssensoren 5 zur Bestimmung der Rotorposition heranzuziehen.

Ferner zeigt Fig. 5 noch drei weitere Sensoren 6, die für die Bestimmung der jeweils aktuellen Drehstellung des Rotors 2 verwendet werden. Mit ihnen lässt sich der aktuelle Drehwinkel (gemessen gegen einen beliebig festlegbaren Nullwinkel) des Rotors 2 relativ zum Stator 3 bestimmen. Dieser Drehwinkel wird in der Regel für die Regelung des elektromagnetischen Drehantriebs 1 bei einer Ausgestaltung nach dem Prinzip des lagerlosen Motors benötigt. Die drei Sensoren 6 sind beispielsweise auf einem darstellungsgemäss unterhalb des Stators angeordneten Elektronikprint 61 angeordnet. Alle drei Sensoren 6 sind in axialer Richtung gesehen in der gleichen Lücke zwischen zwei benachbarten unteren Polen 320 des unteren Statorteils 32 angeordnet und haben alle den gleichen Abstand von der Solldrehachse A. Die Sensoren 6 sind vorzugsweise als Hallsensoren oder als Wirbelstromsensoren ausgestaltet, wobei beispielsweise beim Vorhandensein eines metallischen Spaltrohres zwischen dem Rotor 2 und dem Stator 3 Hallsensoren bevorzugt sind. Je nach Ausgestaltung reicht das magnetische Streufeld am Ort der Sensoren 6 aus, um den jeweils aktuellen Wert des Drehwinkels des Rotors 2 zu ermitteln. Falls dieses Streufeld nicht ausreichend ist, kann jeder der Hallsensoren 6 jeweils mit einem kleinen Permanentmagneten (nicht dargestellt) ausgestattet sein, der beispielsweise auf den jeweiligen Sensor 6 aufgeklebt wird.

Es versteht sich, dass die hier beschriebene Anordnung zur Bestimmung des aktuellen Drehwinkels des Rotors 2 mit den Sensoren 6 in gleicher oder sinngemäss gleicher Weise auch bei allen anderen Varianten und bei allen anderen Ausführungsbeispielen realisiert sein kann.

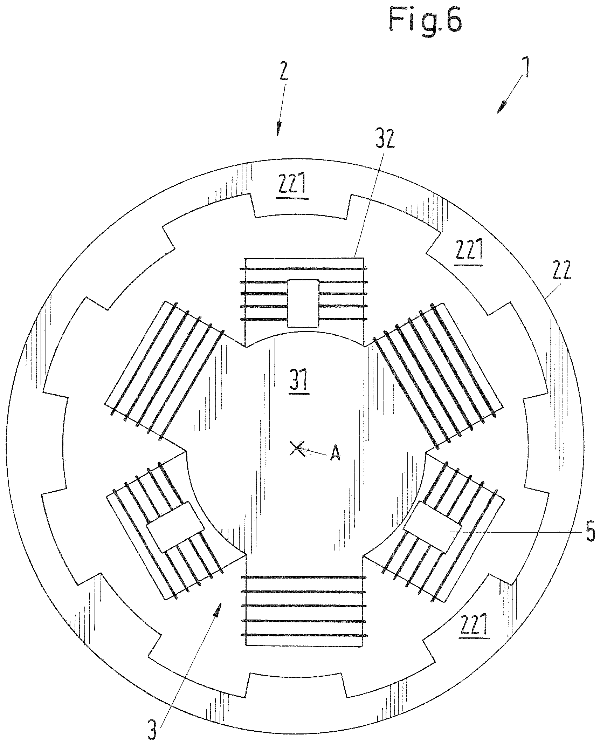

Fig. 6 zeigt eine Aufsicht auf den Stator 3 und den magnetisch wirksamen Kern 22 des Rotors 2 aus Richtung der Solldrehachse A für eine zweite Variante des ersten Ausführungsbeispiels mit einer alternativen Ausgestaltung des magnetisch wirksamen Kerns 22 des Rotors 2. Bei dieser Variante weist der magnetisch wirksame Kern 22 des Rotors 2 insgesamt acht Rotorpole 221 auf, die äquidistant über den inneren Umfang des magnetisch wirksamen Kerns 22 des Rotors 2 verteilt sind und die alle die gleiche in Umfangsrichtung gemessene Länge aufweisen. Auch bei dieser Variante kann der magnetisch wirksame Kern 22 des Rotors den in Fig. 5 dargestellten umlaufenden Ring 222 aufweisen.

Die Erhöhung der Anzahl der Rotorpole 221 auf eine Zahl, welche nicht einem Vielfachen der Anzahl der Pole 310 bzw. 320 des Stators 3 entspricht, hat den Vorteil, dass sich insbesondere bei solchen Ausgestaltungen des elektromagnetischen Drehantriebs 1, die eine Einphasencharakteristik aufweisen, die Anzahl der möglichen Einrastpositionen, in denen kein resultierendes Drehmoment vom Stator 3 auf den Rotor 2 ausgeübt werden kann, deutlich vermindern oder sogar auf Null gebracht werden kann. Das Problem der Einrast- oder Rastpositionen (oder Rastmomente) ist insbesondere aus der Technik der Einphasenmotoren bekannt. Es gibt bestimmte relative Winkelstellungen zwischen Stator 3 und Rotor 2, in denen sich mit dem Stator 3 kein resultierendes Drehmoment auf den Rotor 2 bewirken lässt. Fällt eine solche Winkelstellung mit einer Rastposition, also einer Position, bei welcher das Nutrastmoment ein Maximum aufweist, zusammen so kann der Motor nicht mehr selbständig anlaufen.

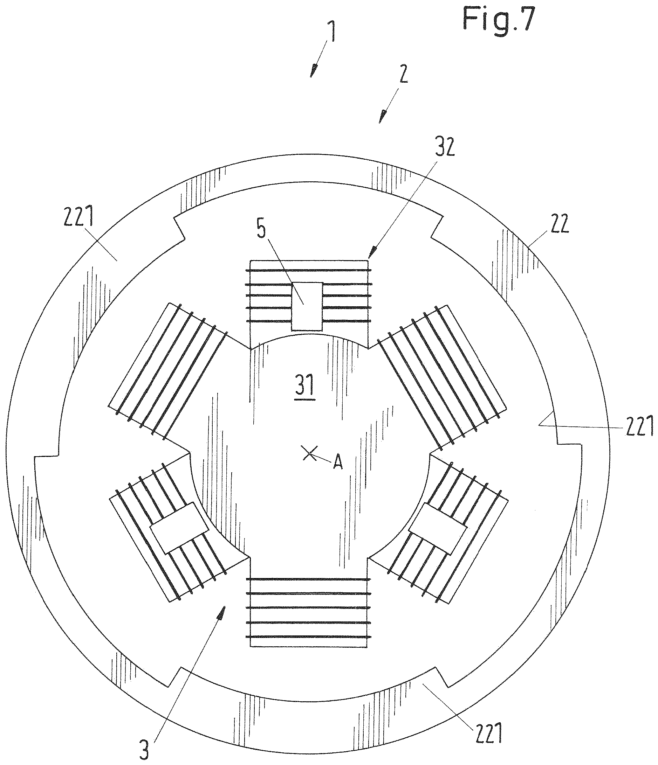

Fig. 7 zeigt in einer zu Fig. 6 analogen Darstellung eine Aufsicht auf den Stator 3 und den magnetisch wirksamen Kern 22 des Rotors 2 aus Richtung der Solldrehachse A für eine dritte Variante des ersten Ausführungsbeispiels mit einer alternativen Ausgestaltung des magnetisch wirksamen Kerns 22 des Rotors 2. Bei dieser Variante weist der magnetisch wirksame Kern 22 des Rotors 2 insgesamt drei Rotorpole 221 auf, die äquidistant über den inneren Umfang des magnetisch wirksamen Kerns 22 des Rotors 2 verteilt sind und die alle die gleiche in Umfangsrichtung gemessene Länge aufweisen. Auch bei dieser Variante kann der magnetisch wirksame Kern 22 des Rotors den in Fig. 5 dargestellten umlaufenden Ring 222 aufweisen. Bei Ausführungsformen, bei welchen die Anzahl der Rotorpole 221 mit der Anzahl der oberen 310 bzw. der unteren Pole 320 des Stators 3 übereinstimmt oder einem Vielfachen der Anzahl dieser Pole 310 bzw. 320 entspricht, können in jeder Winkelstellung der Rotors 2 magnetische Radialkräfte erzeugt werden. Auf der andern Seite sind solche Ausführungsformen bezüglich der Drehmomentbildung ungünstig, falls der Drehantrieb Einphasencharakteristik hat, da die Einrastpositionen der Rotors 2 exakt mit den Winkelpositionen übereinstimmen, bei welchen kein Drehmoment erzeugt werden kann.

Um in den Einrastpositionen des Rotors immer ein Anfahrmoment erzeugen zu können, ist es dabei vorteilhaft, wenn die Rotorpole 221 zumindest leicht asymmetrisch ausgestaltet oder angeordnet sind. Um diese Asymmetrie zu erzielen gibt es viele Möglichkeiten, von denen hier nur einige beispielshaft erwähnt werden. So ist es beispielsweise möglich, die Rotorpole 221 nicht genau äquidistant über den Umfang des magnetisch wirksamen Kerns 22 zu verteilen, oder die seitlichen Begrenzungskanten der Rotorpole 221 können asymmetrisch ausgestaltet sein, beispielsweise mit unterschiedlichen Abschrägungen. Auch ist es möglich, dass die einzelnen Rotorpole 221 zumindest leicht unterschiedliche Längen - gemessen in der Umfangsrichtung - aufweisen. Ferner kann die Ausdehnung eines Rotorpols 221 in radialer Richtung über seine in Umfangsrichtung gesehene Länge variieren.

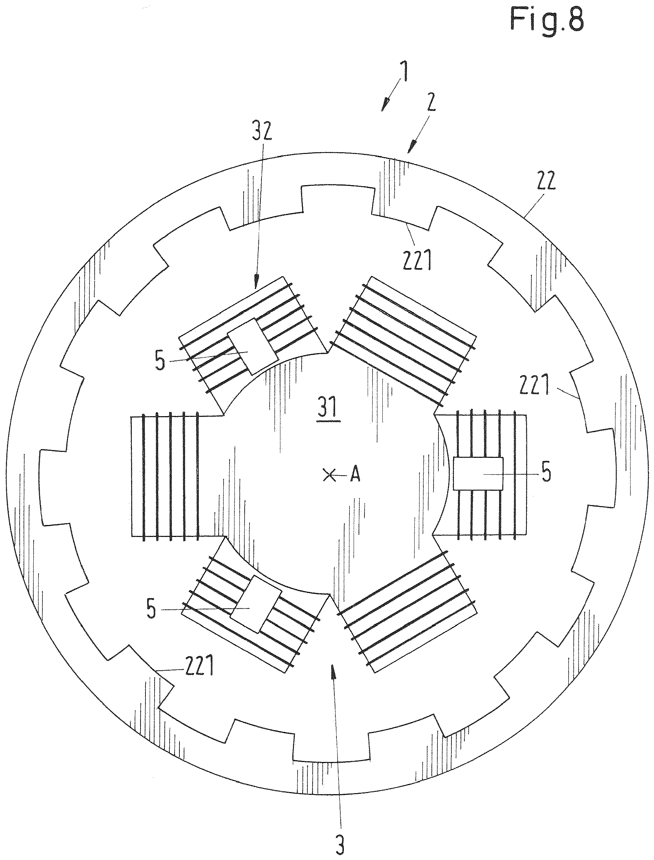

Fig. 8 zeigt in einer zu Fig. 6 analogen Darstellung eine Aufsicht auf den Stator 3 und den magnetisch wirksamen Kern 22 des Rotors 2 aus Richtung der Solldrehachse A für eine vierte Variante des ersten Ausführungsbeispiels mit einer alternativen Ausgestaltung des magnetisch wirksamen Kerns 22 des Rotors 2. Bei dieser Variante weist der magnetisch wirksame Kern 22 des Rotors 2 insgesamt zwölf Rotorpole 221 auf, die äquidistant über den inneren Umfang des magnetisch wirksamen Kerns 22 des Rotors 2 verteilt sind und die alle die gleiche in Umfangsrichtung gemessene Länge aufweisen. Auch bei dieser Variante kann der magnetisch wirksame Kern 22 des Rotors den in Fig. 5 dargestellten umlaufenden Ring 222 aufweisen.

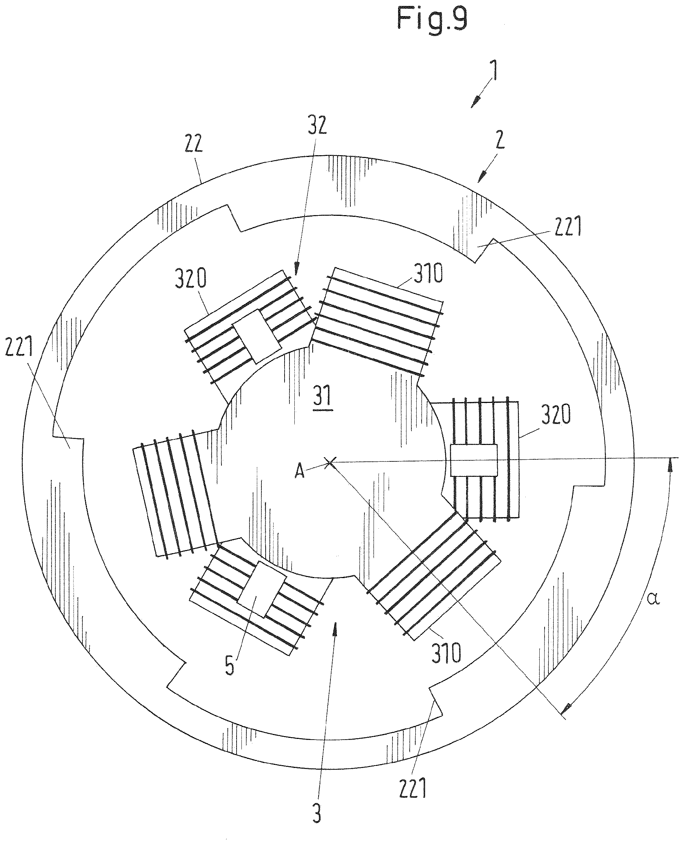

Die vergleichsweise hohe Anzahl von zwölf Rotorpolen 221 ist insbesondere im Hinblick auf die Radialkraftregelung vorteilhaft, da der Einfluss des Rotorwinkels auf die Radialkraftamplitude mit zunehmender Polzahl abnimmt. Die Symmetrie der Anordnung ist bezüglich der Radialkraftregelung zusätzlich von Vorteil. Da die Anzahl Rotorpole 221 aber einem Vielfachen der Anzahl der oberen 310 bzw. der unteren Pole 320 des Stators 3 entspricht und der Drehantrieb gemäss der Ausführungsform in Fig. 8 Einphasencharakteristik aufweist, fallen auch hier die Einrastpositionen mit Rotorwinkeln zusammen, bei welchen sich kein Drehmoment erzielen lässt. Damit der Drehantrieb 1 anfahren kann, sind auch hier zusätzliche Massnahmen vorgesehen. Neben den bereits im Zusammenhang mit Fig. 7 diskutierten Massnahmen, ist auch die im Folgenden anhand von Fig. 9 beschriebene Massnahme auf die Ausführungsform von Fig. 8 anwendbar.

Fig. 9 zeigt eine Aufsicht auf den Stator 3 und den magnetisch wirksamen Kern 22 des Rotors 2 aus Richtung der Solldrehachse A für eine erste Variante des ersten Ausführungsbeispiels mit einer alternativen Ausgestaltung des Stators 3. Bei dieser Variante sind die im Wesentlichen identischen oberen und unteren Statorteile 31 und 32 auch bezüglich der Solldrehachse A relativ zueinander um eine Winkel α verdreht angeordnet, sodass die oberen Pole 310 in axialer Richtung gesehen jeweils zwischen zwei benachbarten unteren Polen 320 angeordnet sind. Jedoch erfüllt der Winkel α bei der in Fig. 9 dargestellten Ausführungsform nicht die Bedingung α = 360°/N, wobei N die Gesamtzahl aller oberen und unteren Pole 310, 320 bezeichnet (hier also N=6), sodass die oberen Pole 310 nicht mehr genau mittig zwischen zwei benachbarten unteren Polen 320 liegen. Dies hat zur Folge, dass in axialer Richtung gesehen, die oberen Pole 310 zwar immer noch in Lücken zwischen zwei benachbarten unteren Polen 320 angeordnet sind, aber sich die oberen 310 und die unteren Pole 320 in axialer Richtung gesehen etwas überlappen. In Fig. 9 beträgt der Winkel α beispielsweise 45°.

Auch dies ist eine vorteilhafte Massnahme, um ein Anfahren aus Einrastpositionen heraus zu gewährleisten, insbesondere wenn der Drehantrieb 1 eine Einphasencharakteristik aufweist, wie beispielsweise in der Ausführungsform gemäss Fig. 9.

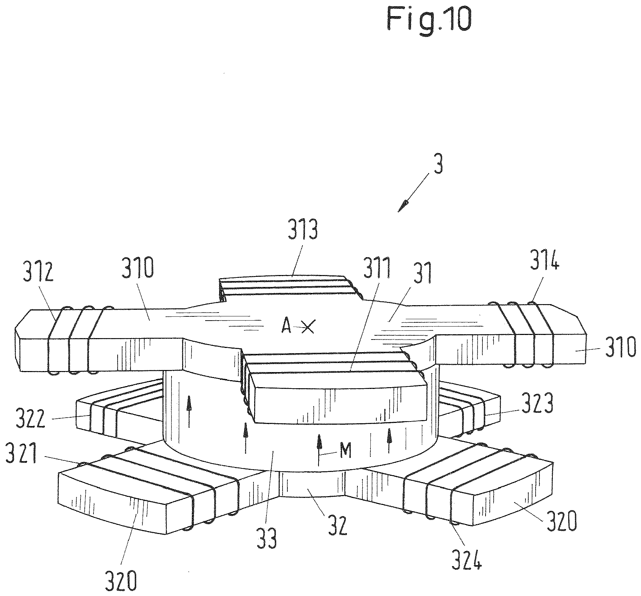

Fig. 10 zeigt in einer perspektivischen Ansicht eine zweite Variante für die Ausgestaltung des Stators 3 des ersten Ausführungsbeispiels. Bei dieser Variante weist der obere Statorteil 31 genau vier obere Pole 310 auf, von denen jeder eine obere Spule 311, 312, 313, 314 als Wicklung trägt. Der untere Statorteil 32 weist genau vier untere Pole 320 auf, von denen jeder eine untere Spule 321, 322, 323, 324 als Wicklung trägt. Auch bei dieser Variante ist also die Anzahl der oberen Pole 310 gleich der Anzahl der unteren Pole 320, wobei hier jedoch die Anzahl der oberen Pole 310 und die Anzahl der unteren Pole 320 jeweils eine gerade Zahl, nämlich vier, ist. Sowohl die oberen Pole 310 als auch die unteren Pole 320 sind jeweils äquidistant bezüglich der Umfangsrichtung angeordnet, das heisst, der Winkel zwischen benachbarten Polen 310, 320 beträgt sowohl im oberen Statorteil 31 als auch im unteren Statorteil 32 jeweils 90°.

Die im Wesentlichen identisch ausgestalteten oberen und unteren Statorteile 31 und 32 sind bei der in den Fig. 10 dargestellten Variante bezüglich der Solldrehachse A relativ zueinander um einen Winkel α = 45° verdreht angeordnet, sodass die oberen Pole 310 in axialer Richtung gesehen jeweils in einer Lücke zwischen zwei benachbarten unteren Polen 320 angeordnet sind. Da hier mit N = 8 die Beziehung α = 360°/N erfüllt ist, ist jeder obere Pol 310 in axialer Richtung betrachtet genau mittig zwischen jeweils zwei benachbarten Polen 320 des unteren Statorteils 32 angeordnet.

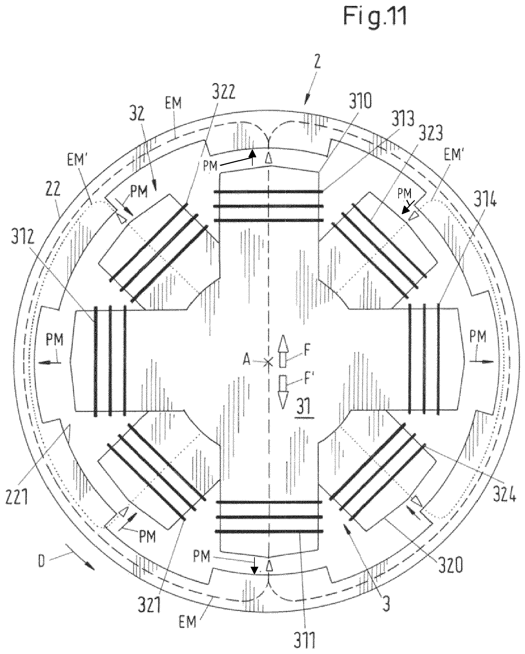

Fig. 11 zeigt eine Aufsicht auf den Stator 3 aus Fig. 10 zusammen mit dem magnetisch wirksamen Kern 22 des Rotors 2, der hier sechs Rotorpole 221 aufweist. Anhand von Fig. 11 soll das Zusammenspiel der beiden Statorteile 31, 32 zur Kraft- und Drehmomenterzeugung auf den Rotor 2 erläutert werden. Da hierfür der permanentmagnetische Fluss unbeachtet bleiben kann, wird im Folgenden nur auf den elektromagnetischen Fluss eingegangen. Fig. 11 zeigt eine Momentaufnahme der Drehposition des magnetisch wirksamen Kerns 22 des Rotors 2 relativ zum Stator 3. Der Verlauf des elektromagnetischen Flusses auf der Ebene des unteren Statorteils 32 ist durch die punktierten Linien mit dem Bezugszeichen EM' angedeutet, der Verlauf des elektromagnetischen Flusses auf der Ebene des oberen Statorteils 31 ist durch die gestrichelten Linien mit dem Bezugszeichen EM angedeutet. Wie dies durch den Verlauf des elektromagnetischen Flusses EM auf der Ebene des oberen Statorteils 31 zu erkennen ist, generiert der obere Statorteil 31 eine resultierende Kraft auf den Stator 3, die durch den Pfeil F angedeutet ist und als Querkraft radial nach aussen gerichtet ist und entsprechend eine gleichgrosse entgegengesetzt gerichtete Kraft F' auf den Rotor 2. Auf dieser Ebene wird jedoch kein resultierendes Drehmoment auf den Rotor ausgeübt. Ebenso ist erkennbar, dass auf der Ebene des unteren Statorteils 32 der elektromagnetische Fluss EM' keine resultierende Kraft auf den Rotor 2 ausübt, aber ein resultierendes Drehmoment auf den Rotor 2 ausgeübt wird, das durch den Pfeil D angedeutet ist. Bei dieser Momentaufnahme generiert also der obere Statorteil die Querkraft F auf den Rotor 2, während der untere Statorteil 32 das Drehmoment D erzeugt.

Hat sich der Rotor 2 um 45° weitergedreht, so kehrt sich die Situation um, dann generiert der untere Statorteil 32 die Kraft auf den Rotor und der obere Statorteil 31 bewirkt das resultierende Drehmoment.

Auch bei der in Fig. 10 bzw. Fig. 11 dargestellten Variante umfasst die Stelleinrichtung zur Ansteuerung des Stators 3 eine Verstärkereinheit 8, die jeweils für jede der acht Spulen 311, 312, 313, 314, 321, 322, 323, 324 einen separaten Leistungsverstärker 81 aufweist, mit welchem der Spulenstrom für jede der Spulen unabhängig von den Spulenströmen der jeweils anderen Spulen regelbar ist.

Wie bereits im Zusammenhang mit Fig. 3 erläutert ist es auch hier möglich, dass jeder separate Leistungsverstärker jeweils als H-Brückenschaltung gemäss Fig. 3 ausgestaltet ist, wobei für die hier beschriebene Variante natürlich dann acht H-Brücken als Leistungsverstärker 81 vorgesehen sind.

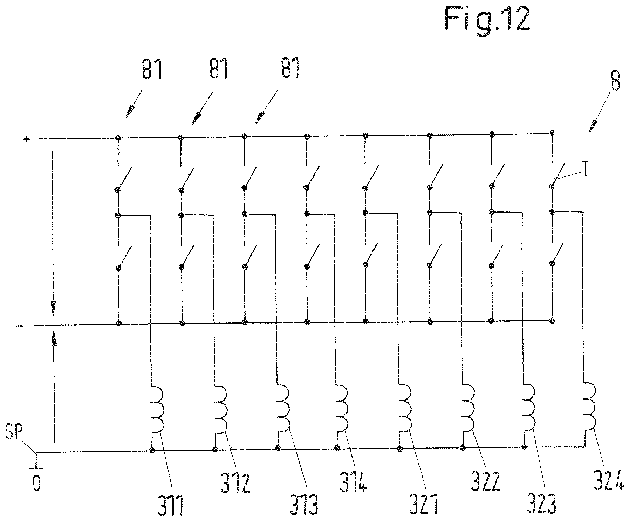

Alternativ kann natürlich auch hier jeder Leistungsverstärker 81 jeweils als ein Brückenzweig einer Verstärkereinheit 8 ausgestaltet sein, in sinngemäss gleicher Weise wie dies im Zusammenhang mit Fig 4 erläutert wurde. Die entsprechende Schaltung ist in Fig. 12 dargestellt. Auch hier ist der Sternpunkt SP vorzugsweise aber nicht zwingend als belastbarer Sternpunkt ausgestaltet.

Bei der in Fig. 10 bzw. Fig. 11 dargestellten Variante ist es auch möglich, jeweils zwei Spulen zu einer elektrischen Phase zusammenzuschalten, wodurch sich die Anzahl der benötigten Leistungsverstärker reduziert, weil nur für jede elektrische Phase ein separater Leistungsverstärker 81 benötigt wird.

Vorzugsweise werden jeweils in dem oberen und in dem unteren Statorteil 31, 32 die sich jeweils gegenüberliegenden Spulen paarweise zusammengeschaltet. Aufgrund der Symmetrie sind dann die Spulenströme in den beiden ein Paar bildenden Spulen umgekehrt gleich gross.

Es werden also die folgenden Spulenpaare jeweils zu einer elektrischen Phase zusammengeschaltet: In dem unteren Statorteil 32 wird die Spule 321 mit der Spule 323 zusammengeschaltet und die Spule 322 mit der Spule 324. In dem oberen Statorteil 31 wird die Spule 311 mit der Spule 313 zusammengeschaltet und die Spule 312 mit der Spule 314.

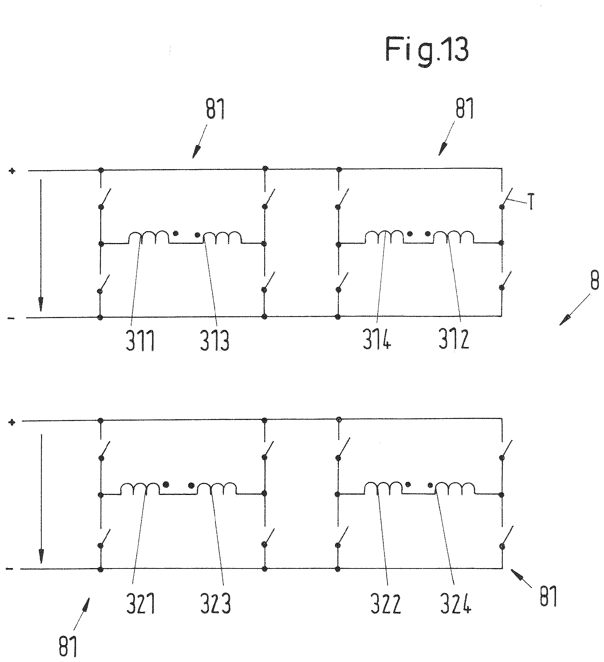

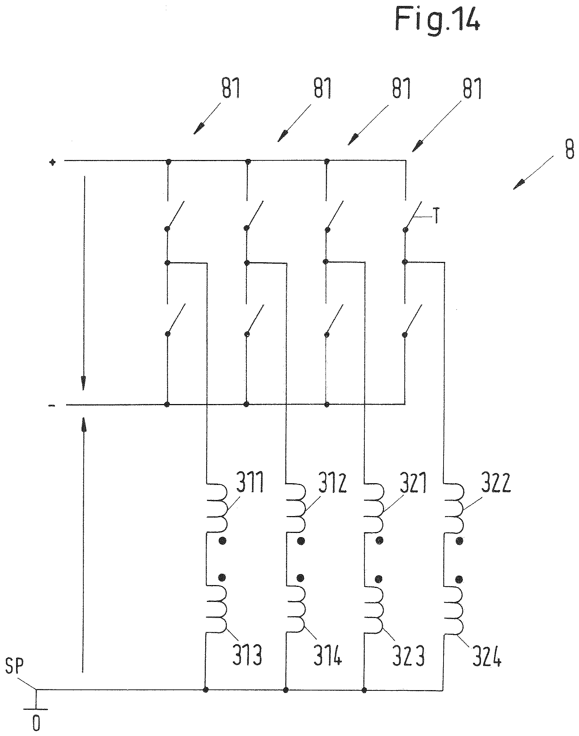

Das entsprechend Schaltbild mit den separaten Leistungsverstärken 81 für jede elektrische Phase ist in Fig. 13 für die Variante mit H-Brückenschaltungen für die separaten Leistungsverstärker 81 dargestellt und in Fig. 14 für die Variante mit Brückenzweigen. Bei der in Fig. 14 gezeigten Schaltung ist es nun aber notwendig, dass der Sternpunkt SP belastbar ist.

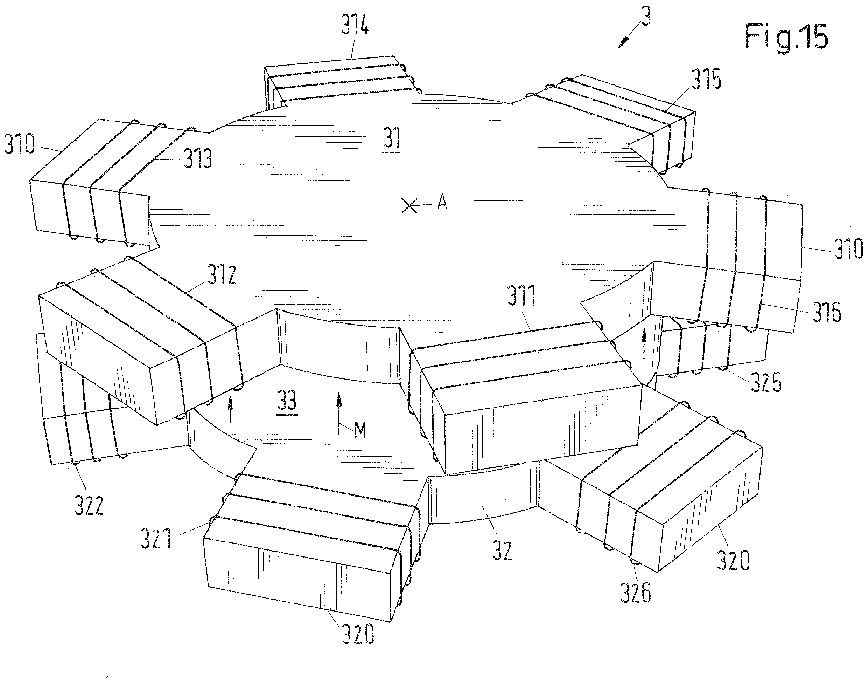

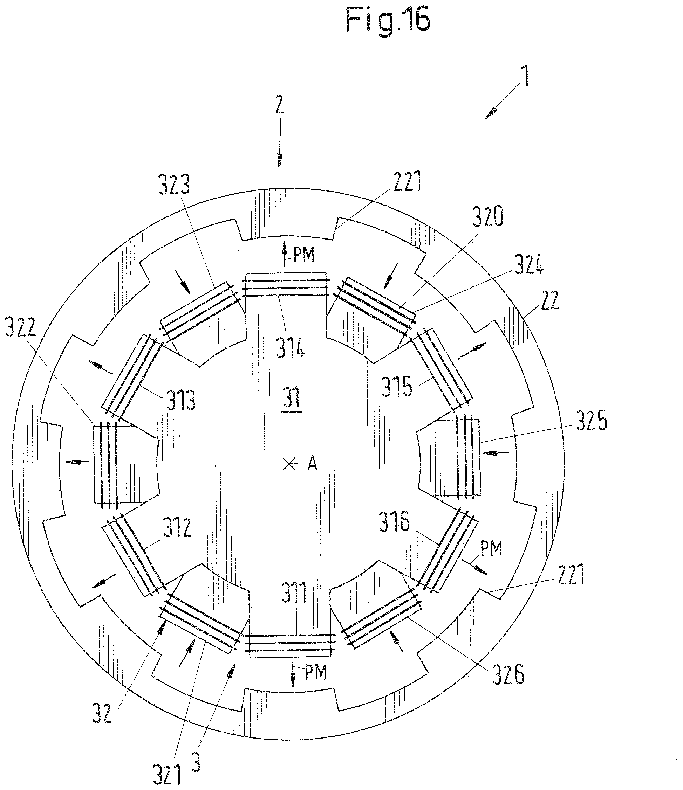

Fig. 15 zeigt in einer perspektivischen Ansicht eine dritte Variante für die Ausgestaltung des Stators 3 des ersten Ausführungsbeispiels. Zum besseren Verständnis zeigt Fig. 16 noch eine Aufsicht aus axialer Richtung auf den Stator 3 aus Fig. 15 zusammen mit dem magnetisch wirksamen Kern 22 des Rotors 2.

Bei dieser Variante weist der obere Statorteil 31 genau sechs obere Pole 310 auf, von denen jeder eine obere Spule 311, 312, 313, 314, 315, 316 als Wicklung trägt. Der untere Statorteil 32 weist genau sechs untere Pole 320 auf, von denen jeder eine untere Spule 321, 322, 323, 324, 325, 326 als Wicklung trägt. Auch bei dieser Variante ist also die Anzahl der oberen Pole 310 gleich der Anzahl der unteren Pole 320, wobei hier die Anzahl der oberen Pole 310 und die Anzahl der unteren Pole 320 jeweils eine gerade Zahl, nämlich sechs, ist. Sowohl die oberen Pole 310 als auch die unteren Pole 320 sind jeweils äquidistant bezüglich der Umfangsrichtung angeordnet, das heisst, der Winkel zwischen benachbarten Polen 310, 320 beträgt sowohl im oberen Statorteil 31 als auch im unteren Statorteil 32 jeweils 60°.

Die im Wesentlichen identisch ausgestalteten oberen und unteren Statorteile 31 und 32 sind bei der in den Fig. 15 und Fig. 16 dargestellten Variante bezüglich der Solldrehachse A relativ zueinander um eine Winkel α = 30° verdreht angeordnet, sodass die oberen Pole 310 in axialer Richtung gesehen jeweils in einer Lücke zwischen zwei benachbarten unteren Polen 320 angeordnet sind. Da hier mit N = 12 die Beziehung α = 360°/N erfüllt ist, ist jeder obere Pol 310 in axialer Richtung betrachtet genau mittig zwischen jeweils zwei benachbarten Polen 320 des unteren Statorteils 32 angeordnet.

Auch bei der in Fig. 15 bzw. Fig. 16 dargestellten Variante umfasst die Stelleinrichtung zur Ansteuerung des Stators 3 eine Verstärkereinheit 8, die jeweils für jede der zwölf Spulen 311, 312, 313, 314, 315, 316, 321, 322, 323, 324, 325, 326 einen separaten Leistungsverstärker 81 aufweist, mit welchem der Spulenstrom für jede der Spulen unabhängig von den Spulenströmen der jeweils anderen Spulen regelbar ist.

Wie bereits im Zusammenhang mit Fig. 3 erläutert ist es auch hier möglich, dass jeder separate Leistungsverstärker jeweils als H-Brückenschaltung gemäss Fig. 3 ausgestaltet ist, wobei für die hier beschriebene Variante natürlich dann zwölf H-Brücken als Leistungsverstärker 81 vorgesehen sind.

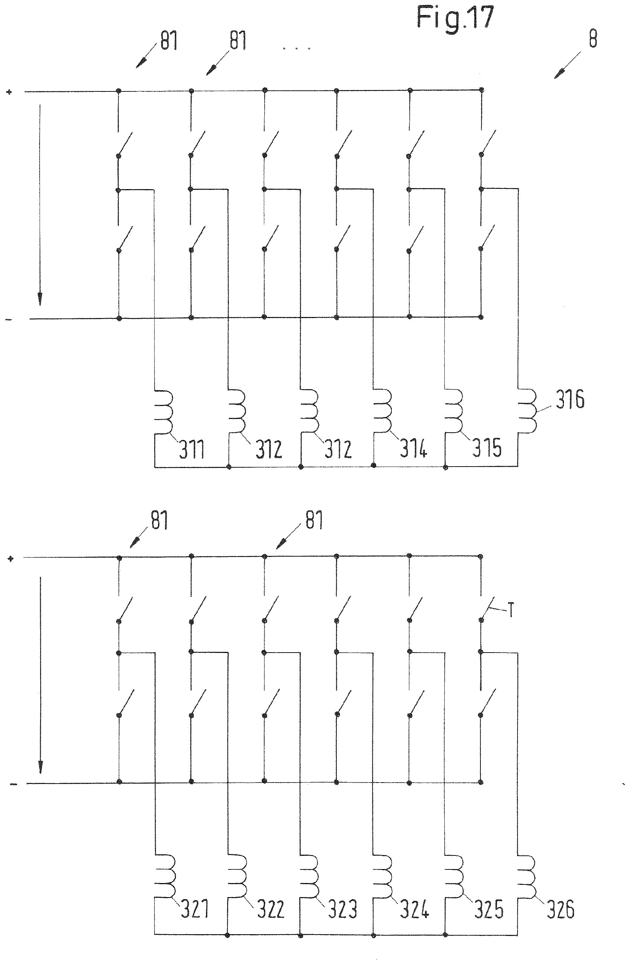

Alternativ kann natürlich auch hier jeder Leistungsverstärker 81 jeweils als ein Brückenzweig einer Verstärkereinheit 8 ausgestaltet sein, in sinngemäss gleicher Weise wie dies im Zusammenhang mit Fig. 4 erläutert wurde. Die entsprechende Schaltung ist in Fig. 17 dargestellt. Auch hier ist der Sternpunkt SP vorzugsweise aber nicht zwingend als belastbarer Sternpunkt ausgestaltet.

Auch bei der in Fig. 15 bzw. Fig. 16 dargestellten Variante ist es möglich, jeweils zwei Spulen zu einer elektrischen Phase zusammenzuschalten, wodurch sich die Anzahl der benötigten Leistungsverstärker reduziert, weil nur für jede elektrische Phase ein separater Leistungsverstärker 81 benötigt wird.

Vorzugsweise werden jeweils in dem oberen und in dem unteren Statorteil 31, 32 die sich jeweils gegenüberliegenden Spulen paarweise zusammengeschaltet. Es werden also die folgenden Spulenpaare jeweils zu einer elektrischen Phase zusammengeschaltet: In dem unteren Statorteil 32 wird die Spule 321 mit der Spule 324 zusammengeschaltet, die Spule 322 mit der Spule 325 und die Spule 323 mit der Spule 326. In dem oberen Statorteil 31 wird die Spule 311 mit der Spule 314 zusammengeschaltet, die Spule 312 mit der Spule 315, und die Spule 313 mit der Spule 316.

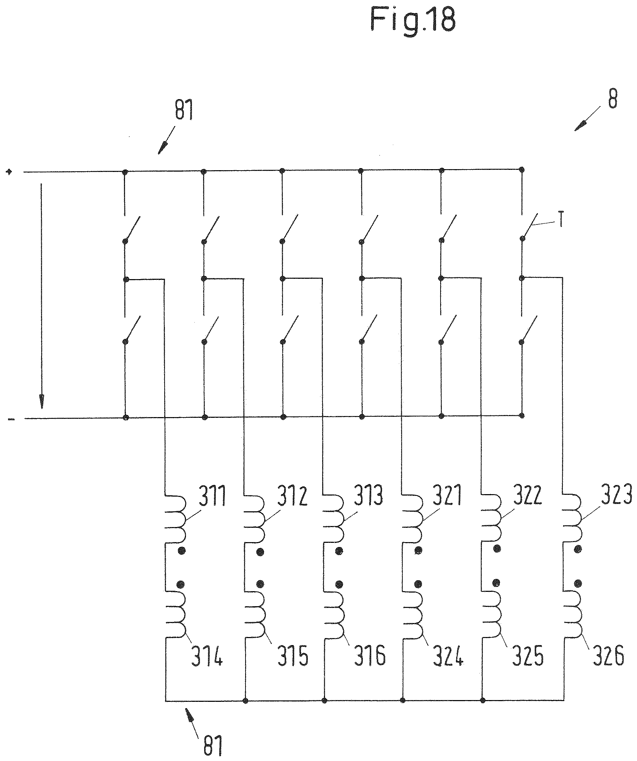

Das entsprechende Schaltbild mit den separaten Leistungsverstärken 81 für jede elektrische Phase ist in Fig. 18 für die Variante mit Brückenzweigen dargestellt. Bei der in Fig. 18 gezeigten Schaltung ist es vorteilhaft, aber nicht notwendig, dass der Sternpunkt SP belastbar ist.

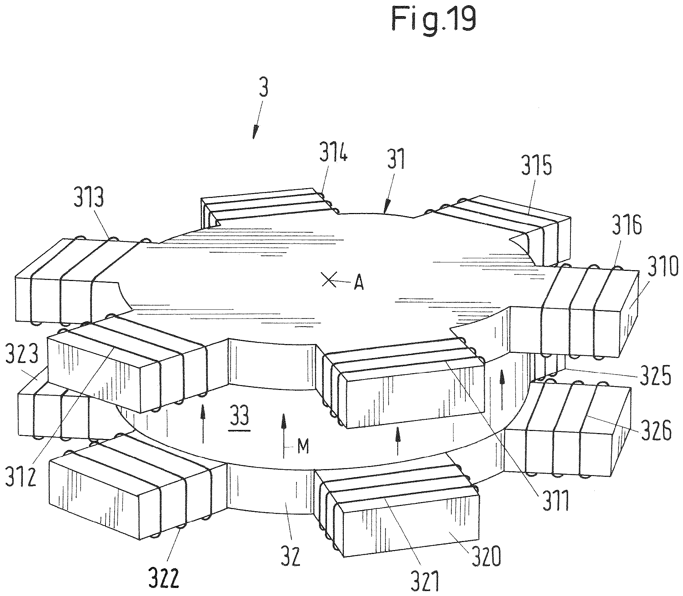

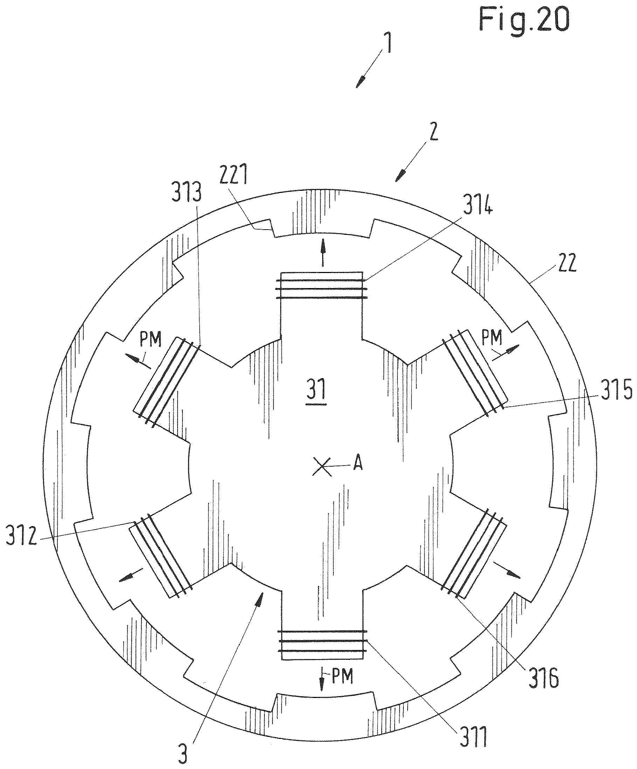

Fig. 19 zeigt in einer perspektivischen Ansicht eine vierte Variante für die Ausgestaltung des Stators 3 des ersten Ausführungsbeispiels. Zum besseren Verständnis zeigt Fig. 20 noch eine Aufsicht aus axialer Richtung auf den Stator 3 aus Fig. 19 zusammen mit dem magnetisch wirksamen Kern 22 des Rotors 2.

Auch bei dieser Variante weist der obere Statorteil 31 genau sechs obere Pole 310 auf, von denen jeder eine obere Spule 311, 312, 313, 314, 315, 316 als Wicklung trägt. Der untere Statorteil 32 weist genau sechs untere Pole 320 auf, von denen jeder eine untere Spule 321, 322, 323, 324, 325, 326 als Wicklung trägt. Auch bei dieser Variante ist also die Anzahl der oberen Pole 310 gleich der Anzahl der unteren Pole 320, wobei die Anzahl der oberen Pole 310 und die Anzahl der unteren Pole 320 jeweils eine gerade Zahl, nämlich sechs, ist. Sowohl die oberen Pole 310 als auch die unteren Pole 320 sind jeweils äquidistant bezüglich der Umfangsrichtung angeordnet, das heisst, der Winkel zwischen benachbarten Polen 310, 320 beträgt sowohl im oberen Statorteil 31 als auch im unteren Statorteil 32 jeweils 60°.

Die im Wesentlichen identisch ausgestalteten oberen und unteren Statorteile 31 und 32 sind bei der in den Fig. 19 und Fig. 20 dargestellten Variante bezüglich der Solldrehachse A nicht relativ zueinander verdreht, das heisst der Winkel α ist gleich 0°. Folglich sind die oberen Pole 310 und die unteren Pole 320 so angeordnet, dass sie sich in axialer Richtung gesehen überdecken. Bei dieser Ausgestaltung des Stators 3 ist es möglich, den Rotor 3 auch gegen Verkippungen gegen die radiale Ebene aktiv magnetisch zu stabilisieren, sodass nun diese beiden Freiheitsgrade der Verkippung auch aktiv magnetisch geregelt werden können. Dazu ist es jedoch notwendig, dass für jede der insgesamt zwölf Spulen der Spulenstrom jeweils unabhängig von dem Spulenstrom für die anderen Spulen regelbar ist.

Auch bei der in Fig. 19 bzw. Fig. 20 dargestellten Variante ist es möglich, jeweils zwei Spulen zu einer elektrischen Phase zusammenzuschalten, wodurch sich die Anzahl der benötigten Leistungsverstärker reduziert, weil nur für jede elektrische Phase ein separater Leistungsverstärker 81 benötigt wird. Neben der vorangehend beschriebenen Zusammenschaltung von jeweils zwei Spulen des oberen oder des unteren Statorteils 31, 32, ist es bei der in Fig. 19 und Fig. 20 dargestellten Variante auch möglich, jeweils eine Spule des unteren Statorteils 32 mit einer Spule des oberen Statorteils 31 zusammenzuschalten. Dabei werden vorzugsweise die jeweils bezüglich der axialen Richtung übereinanderliegenden Spulen zusammengeschaltet. Es werden dann also die Spule 311 mit der Spule 321 zusammengeschaltet, die Spule 312 mit der Spule 322, die Spule 313 mit der Spule 323, die Spule 314 mit der Spule 324, die Spule 315 mit der Spule 325, und die Spule 316 mit der Spule 326.

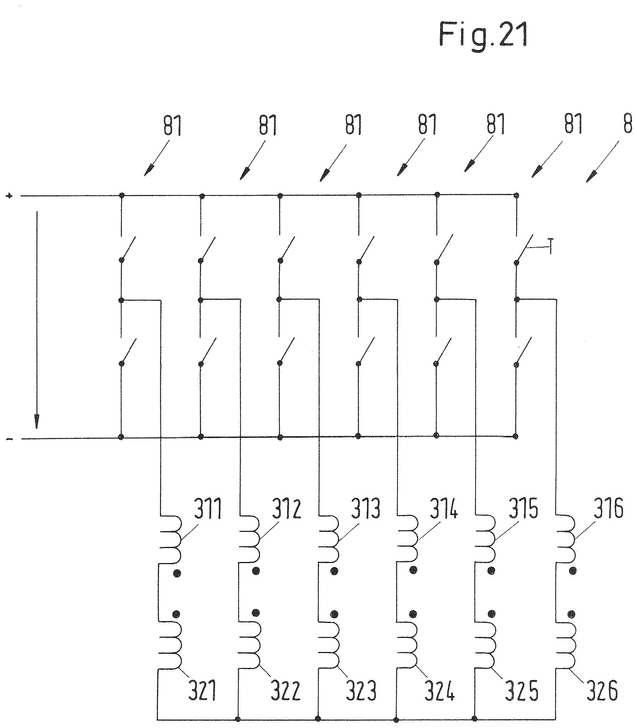

Das entsprechend Schaltbild mit den separaten Leistungsverstärken 81 für jede elektrische Phase ist in Fig. 21 für die Variante mit Brückenzweigen dargestellt. Bei der in Fig. 21 gezeigten Schaltung ist es vorteilhaft, aber nicht notwendig, dass der Sternpunkt SP belastbar ist.

Beim Zusammenschalten von jeweils zwei Spulen ist vorangehend nur eine Zusammenschaltung der Spulen in Serie beschrieben. Es versteht sich jedoch, dass die Spulen auch in einer Parallelschaltung zusammengeschaltet werden können.

Fig. 22 zeigt in einer perspektivischen Schnittdarstellung ein zweites Ausführungsbeispiel des erfindungsgemässen Drehantriebs 1, welches als Pump- oder Mischvorrichtung ausgestaltet ist. Im Folgenden wird nur auf die Unterschiede zu dem vorangehend beschriebenen ersten Ausführungsbeispiel eingegangen. Insbesondere haben die Bezugszeichen die gleiche Bedeutung wie sie bereits im Zusammenhang mit dem ersten Ausführungsbeispiel erläutert sind. Es versteht sich, dass alle vorangehend beschriebenen Varianten, Ausführungsformen und Massnahmen in gleicher Weise oder in sinngemäss gleicher Weise auch bei dem zweiten Ausführungsbeispiel realisiert sein können.

Derartige Ausgestaltungen des erfindungsgemässen Drehantriebs 1 als Pump- oder Mischvorrichtung können beispielsweise in der pharmazeutischen Industrie und in der biotechnologischen Industrie Verwendung finden. Speziell eignet sich diese Ausgestaltung für solche Anwendungen, bei denen ein sehr hohes Mass an Reinheit oder Sterilität derjenigen Komponenten wesentlich ist, die mit den zu mischenden Substanzen in Kontakt kommen. Diese Ausgestaltung des erfindungsgemässen Drehantriebs 1 kann auch als Bioreaktor oder als Fermenter ausgebildet sein. Es versteht sich jedoch, dass diese Ausgestaltung auch ganz allgemein eine Pump- oder Mischvorrichtung sein kann, mit der Medien oder Substanzen gemischt werden. Insbesondere können diese Substanzen Fluide oder Feststoffe, vorzugsweise Pulver, sein. Derartige Pump- oder Mischvorrichtungen eignen sich zum Mischen von Flüssigkeiten untereinander und/oder zum Mischen von mindestens einer Flüssigkeit mit einem Pulver oder sonstigen Feststoff und/oder zum Mischen von Gasen mit Flüssigkeiten und/oder Feststoffen.

Bei dem in Fig. 22 dargestellten zweiten Ausführungsbeispiel umfasst der als Pump- oder Mischvorrichtung ausgestaltete elektromagnetische Drehantrieb 1 einen flexiblen Mischbehälter 71 zur Aufnahme der zu mischenden Substanzen, welcher aus einem Kunststoff hergestellt ist und welcher in Fig. 22 nur angedeutet ist. Der Mischbehälter 71 ist vorzugsweise ein flexibler Beutel, beispielsweise ein Plastik- oder ein Kunststoffsack, der zusammengefaltet werden kann, sodass er bei der Lagerung möglichst wenig Platz beansprucht. Der Mischbehälter 71 ist in einen in Fig. 22 ebenfalls nur angedeuteten Stützbehälter 51 eingelegt, welcher den Mischbehälter 71 stützt.

Der Mischbehälter 71 weist in seinem Bodenbereich einen im wesentlichen zylindrischen Becher 75 auf, der sich in den Innenraum des Mischbehälters 71 erstreckt und im Zentrum des Bodenbereichs angeordnet ist. Der zylindrische Becher 75 ist vorzugsweise formstabil und aus einem Kunststoff gefertigt. Er kann beispielsweise auch in Form eines flexiblen Schlauches ausgestaltet sein.

In dem Mischbehälter 71 ist der Rotor 2 angeordnet, welcher ein Flügelrad 21 mit mehreren Flügeln zum Mischen der Substanzen umfasst. Damit der Rotor 2 berührungslos magnetisch antreibbar - also in Rotation versetzbar - ist, umfasst er den magnetisch wirksamen Kern 22 (siehe hierzu auch Fig. 1 und Fig. 2), der radial innenliegend bezüglich des Flügelrads 21 angeordnet ist. Der Rotor 2 hat eine im wesentlichen ringförmige Ausgestaltung mit einer zentralen Öffnung, sodass er in der Gebrauchslage, die in Fig. 22 dargestellt ist, um den Becher 75 herum angeordnet ist, welcher sich dann in der zentralen Öffnung des Rotors 2 befindet.

An seiner Oberseite ist der im wesentlichen zylindrisch ausgestaltete Stützbehälter 51 offen, sodass der Mischbehälter 71 problemlos in den Stützbehälter 51 eingebracht werden kann.

An seinem Boden 53 weist der Stützbehälter 51 einen zentral angeordneten, im wesentlichen zylindrisch ausgestalteten Spalttopf 4 zur Aufnahme des Stators 3 auf. Der Spalttopf 4 erstreckt sich in Richtung seiner Zylinderachse, die üblicherweise mit der Solldrehachse A übereinstimmt, darstellungsgemäss nach oben, sodass er im zusammengesetzten Zustand koaxial in dem Becher 75 des Mischbehälters 71 angeordnet ist. Die Abmessungen des Spalttopfs 4 und des Bechers 75 sind dabei so aufeinander abgestimmt, dass der Becher 75 den Spalttopf 4 im zusammengesetzten Zustand eng umschliesst und mit seiner Mantelfläche an der Mantelfläche des Spalttopfs 4 anliegt.

In dem Spalttopf 4 ist der Stator 3 angeordnet, mit welchem der Rotor 2 im Betriebszustand berührungslos um die Solldrehachse A antreibbar und bezüglich des Stators 3 berührungslos magnetisch lagerbar ist.

Das Zusammensetzen des Mischbehälters 71 mit dem darin enthaltenen Rotor 3 und des Stützbehälters 51. ist äusserst einfach, sowie schnell und insbesondere ohne Werkzeuge durchführbar. Dazu wird der üblicherweise für die Lagerung zusammengefaltete Mischbehälter 71 mit dem darin befindlichen Rotor 2 seiner Verpackung entnommen, in den Stützbehälter 51 eingelegt und der Becher 75 mit dem darumliegenden Rotor 2 über den Spalttopf 4 gestülpt. Schon dann ist der als Pump- oder Mischvorrichtung ausgestaltete Drehantrieb 1 bereit für die Anwendung. Nach der Anwendung wird der Mischbehälter 71 mit dem Becher 75 und dem Rotor 2 einfach aus dem Stützbehälter 51 herausgezogen. Der Becher 75 löst sich dabei einfach vom Spalttopf 4 ab. Diese besonders einfache und problemlose Verbindung bzw. Trennung macht dieses zweite Ausführungsbeispiel insbesondere auch für den Einmalgebrauch verwendbar, wobei dann der Mischbehälter 71 und der Rotor 2 für den Einmalgebrauch ausgelegt sein können, während der Stützbehälter 51 und der Stator 3 mit dem Spalttopf 4 für den Dauergebrauch bzw. für den Mehrfachgebrauch ausgelegt sind.

Der Stator 3 ist bei diesem Ausführungsbeispiel mittels einer thermisch leitfähigen Vergussmasse im Spalttopf 4 eingegossen und somit im Spalttopf 4 fixiert. Der Spalttopf 4 ist darstellungsgemäss unten durch einen Topfboden 41 abgeschlossen, welcher mehrere Kühlrippen 42 aufweist. Der Topfboden 41 umfasst einen radial aussenliegenden Flansch 43, welcher der Befestigung des Spalttopfs 4 am Boden 53 des Stützbehälters 51 dient. Der Boden 53 weist eine zentral angeordnete kreisförmige Öffnung 531 auf, welche so bemessen ist, dass der Spalttopf 4 darstellungsgemäss von unten durch die Öffnung 531 in den Innenraum des Stützbehälters 51 eingeschoben werden kann und dann mittels Schrauben 44, die durch den Flansch 43 hindurchgreifen, am Boden 53 des Stützbehälters 51 fixiert werden kann. Der Topfboden 41 weist eine Bohrung auf, durch welche sich eine Leitung 45 in den Innenraum des Spalttopfes 4 erstreckt. In der Leitung 45 sind sämtliche elektrischen Verbindungen zusammengefasst, welche für die Energieversorgung und die Ansteuerung des Stators 3 sowie für den Datenaustausch zwischen Sensoren und Messeinrichtungen mit der nicht dargestellten Kontroll- und Regeleinrichtung notwendig sind. Diese elektrischen Verbindungen sind gesamthaft mit dem Bezugszeichen 46 versehen. Der Spalttopf 4 kann aus einem metallischen Material oder aus einem Kunststoff gefertigt sein.

Eine Variante zu der fixen Verbindung zwischen dem Spalttopf 4 und dem Stützbehälter 51 besteht darin, dass der Spalttopf 4 erst nach dem Einlegen des Mischbehälters 71 darstellungsgemäss von unten in den Becher 75 eingeführt wird. Dieses Einführen kann sowohl von Hand erfolgen oder aber auch durch eine Hubeinrichtung, welche den Spalttopf 4 durch die Öffnung 531 in den Becher 75 einfährt und ihn dann in dieser Position hält. Bei dieser Variante ist keine gesonderte Fixierung des Spalttopfs 4 am Stützbehälter 51 notwendig. Nach der Anwendung fährt die Hubeinrichtung den Spalttopf 4 dann wieder darstellungsgemäss (Fig. 22) nach unten.

Da der Becher 75 des Mischbehälters 71 vorzugsweise formstabil ausgestaltet ist, der Rest des als Beutel ausgestalteten Mischbehälters 71 aber üblicherweise nicht, ist es vorteilhaft, jedoch nicht zwingend notwendig den Becher 75 als separates Teil herzustellen und ihn anschliessend mit dem Mischbehälter 71 zu verbinden. Eine Möglichkeit hierzu ist insbesondere auch in Fig. 22 dargestellt. Der formstabile im Wesentlichen zylindrische Becher 75 wird als separates Teil mit einem an seinem darstellungsgemäss unteren Ende -also an dem offenen Ende - entlang des Umfangs verlaufenden, sich in radialer Richtung ersteckenden Flansch 751 hergestellt, beispielsweise mit einem Spritzgiessverfahren. Der Rest des als Beutel ausgestalteten Mischbehälters 71 weist eine kreisförmige Öffnung auf, deren Durchmesser kleiner ist als der Durchmesser des Flansches 751. Der Becher 75 wird dann in den Mischbehälter 71 eingeführt, sodass der Rand 711, welcher die kreisförmige Öffnung begrenzt, auf dem Flansch 751 des Bechers 75 aufliegt und mit diesem überlappt. Anschliessend wird der Becher 75 im Bereich des Überlapps zwischen dem Flansch 751 und dem Rand 711 mit dem Mischbehälter verschweisst oder verklebt, sodass die Schweiss- oder Klebenaht den Becher 75 fest und unlösbar mit dem Mischbehälter 71 verbindet.

An der äusseren Mantelfläche des Bechers 75 sind vorzugsweise eine Mehrzahl von oberen Halteelementen 753 und unteren Halteelementen 752 über den Umfang des Bechers 75 verteilt angeordnet. In der dargestellten Variante sind je vier obere und untere Halteelemente 753 bzw. 752 vorgesehen. Jedes Halteelement 752, 753 ist jeweils als ein Noppen ausgestaltet, der sich in radialer Richtung von der Solldrehachse A weg erstreckt. Dabei sind je ein oberes Halteelement 753 und ein unteres Halteelement 752 paarweise so angeordnet, dass sie bezüglich der axialen Richtung miteinander fluchten und um eine Distanz B voneinander entfernt sind. Die Distanz B ist dabei so gewählt, dass der Rotor 2 bezüglich der axialen Richtung mit deutlichem Spiel zwischen die oberen und die unteren Halteelemente 753 und 752 passt. Die Länge der Halteelemente 752 und 753 in radialer Richtung ist so bemessen, dass die Bewegungsmöglichkeit des Rotors 2 in axialer Richtung durch die Halteelemente 753 bzw. 752 beschränkt wird. Der Rotor 2 kann sich also in axialer Richtung lediglich zwischen den Halteelementen 753 und 752 bewegen. Diese Massnahme ist insbesondere für die Lagerung des Mischbehälters 75 vor der Benutzung und für das Einsetzten des Mischbehälters 75 mit dem darin befindlichen Rotor 2 in den Stützbehälter 51 vorteilhaft, weil der Rotor 2 somit ungefähr in der Lage gehalten wird, die er während des Betriebs einnehmen soll. Während des Betriebs der Mischvorrichtung 1 haben die Halteelemente 753, 752 keine Funktion.

Der Rotor 2 umfasst den magnetisch wirksamen Kern 22 sowie das Flügelrad 21 und ist spulenfrei ausgestaltet, d. h. auf dem Rotor 2 sind keine Wicklungen vorgesehen. In einer ganz besonders bevorzugten Ausgestaltung, die auch in dem hier beschriebenen Ausführungsbeispiel realisiert ist, weist der Rotor 2 bzw. der magnetisch wirksame Kern 22 des Rotors 2 keine Permanentmagnete auf, er ist also frei von Permanentmagneten. Diese Massnahme ermöglicht eine besonders kostengünstige Ausgestaltung des Rotors 2 was insbesondere bei einer Ausgestaltung des Rotors 2 als Einmalteil einen grossen Vorteil darstellt. Denn für die Herstellung des Rotors 2 sind keine Seltenen Erden wie z. B. Neodym oder Samarium, bzw. Verbindungen oder Legierungen dieser notwendig, die häufig für die Herstellung von Permanentmagneten verwendet werden. Der Verzicht auf diese Permanentmagnete bedeutet auch unter Umweltaspekten einen grossen Vorteil.

Der Rotor 2 weist ferner das Flügelrad 21 mit mehreren über den Umfang des Rotors 2 verteilten Flügeln 211 auf, welche die Substanzen in dem Mischbehälter 71 durchmischen können. Das Flügelrad 21 ist radial aussenliegend bezüglich des magnetisch wirksamen Kerns 22 angeordnet, wobei die Flügel 211 im Wesentlichen auf der gleichen Höhe bezüglich der axialen Richtung angeordnet sind wie der magnetisch wirksame Kern 22.

Der Rotor 2 ist als Integralrotor ausgestaltet, weil er sowohl der Rotor 2 des elektromagnetischen Antriebs 1 ist, als auch der Rotor der magnetischen Lagerung, als auch der Rotor 2 des Mischers. Dies bietet den Vorteil einer sehr kompakten und platzsparenden Ausgestaltung Der magnetisch wirksame Kern 22 weist eine Ummantelung 23 auf, die aus einem Kunststoff besteht und den magnetisch wirksamen Kern 22 vollständig umschliesst. Vorzugsweise ist der magnetisch wirksame Kern in einen Kunststoff eingegossen, der die Ummantelung 23 bildet. Die Flügel 211 können beispielsweise auf die Ummantelung 23 aufgesetzt werden und dort mittels einer Klemmverbindung fixiert sein, oder mit der Ummantelung 23 verklebt oder verschweisst sein. Auch ist es möglich, dass die Flügel 211 integraler Bestandteil der Ummantelung 23 sind.

Wenn der Rotor 2 und der Mischbehälter 71 für den Einmalgebrauch ausgelegt sind, sollten die aus Kunststoff gefertigten Teile aus einem möglichst preisgünstigen, handelsüblichen Kunststoff hergestellt werden. Ein weiterer wesentlicher Aspekt bei der Ausgestaltung für den Einmalgebrauch ist es, dass die Einmalteile für gewisse Anwendungsbereiche sterilisierbar sein müssen. Dabei ist es besonders vorteilhaft, wenn die Einmalteile gamma-sterilisierbar sind. Bei dieser Art der Sterilisierung wird das zu sterilisierende Element mit Gamma-Strahlung beaufschlagt. Der Vorteil der Gamma-Sterilisierung, beispielsweise im Vergleich zur Dampfsterilisierung, liegt insbesondere darin, dass die Sterilisierung auch durch die Verpackung hindurch erfolgen kann. Gerade bei Einmalteilen ist es eine gängige Praxis, dass die Teile nach ihrer Herstellung in die für den Versand vorgesehene Verpackung gebracht werden und dann noch eine Zeit lagern, bevor sie an den Kunden ausgeliefert werden. In solchen Fällen erfolgt die Sterilisierung erst kurz vor der Auslieferung an den Kunden durch die Verpackung hindurch, was bei einer Dampfsterilisierung oder anderen Verfahren nicht möglich ist.

Es ist in der Regel nicht notwendig, dass die Einmalteile - wie der Mischbehälter 71 und der Rotor 2 mehr als einmal sterilisierbar sein müssen. Dies ist insbesondere bei der Gamma-Sterilisierung ein grosser Vorteil, weil die Beaufschlagung mit Gamma-Strahlung bei Kunststoffen zu Degradationen führen kann, sodass eine mehrfache Gamma-Sterilisierung den Kunststoff unbrauchbar machen kann.

Da in der Regel bei Einmalteilen auf eine Sterilisierung unter hohen Temperaturen und /oder unter hohem (Dampf-) Druck verzichtet werden kann, können kostengünstigere Kunststoffe eingesetzt werden, beispielsweise solche, die keine hohen Temperaturen aushalten, oder die nicht mehrfach hohen Temperatur- und Druckwerten ausgesetzt werden können.

Unter Berücksichtigung all dieser Aspekte ist es daher bei der Ausgestaltung für den Einmalgebrauch bevorzugt, für die Herstellung der Einmalteile solche Kunststoffe zu verwenden, die zumindest einmal gamma-sterilisierbar sind. Die Materialien sollten dabei gammastabil für eine Dosis von mindestens 40 kGy sein, um eine einmalige Gamma-Sterilisierung zu ermöglichen. Bei der Gamma-Sterilisierung sollten zudem keine giftigen Stoffe entstehen. Zudem ist es bevorzugt, wenn alle Materialien, die mit den zu mischenden bzw. den durchmischten Substanzen in Berührung kommen, USP Class VI Standards erfüllen.

Für die Herstellung des flexiblen Mischbehälters 71 sind beispielsweise folgende Kunststoffe bevorzugt: PolyEthylene (PE), Low Density PolyEthylene (LDPE), Ultra Low Density PolyEthylene (ULDPE), Ethylene Vinyl Acetate (EVA), PolyEthylene Terephthalate (PET), PolyVinylChlorid (PVC), PolyPropylene (PP), PolyUrethan (PU), Silicone

Für die Herstellung des Bechers 75 und die aus Kunststoff bestehenden Teile des Rotors 2, also das Flügelrad 21 und die Ummantelung 23 sind beispielsweise folgende Kunststoffe bevorzugt: PolyEthylene (PE), PolyPropylene (PP), Low Density PolyEthylene (LDPE), Ultra Low Density PolyEthylene (ULDPE), Ethylene Vinyl Acetate (EVA), PolyEthylene Terephthalate (PET), PolyVinylChlorid (PVC), PolyVinyliDene Fluoride (PVDF), Acrylonitrile Butadiene Styrene (ABS), PolyAcryl, PolyCarbonate (PC).

Für den Einmalgebrauch weniger geeignete oder sogar ungeeignete Materialien für die Herstellung der Kunststoffteile sind beispielsweise die unter dem Markennamen Teflon bekannten Materialien Polytetrafluoroethylene (PTFE) und und Perfluoralkoxy-Polymere (PFA). Bei diesen Materialien besteht nämlich bei der Gamma-Sterilisierung die Gefahr, dass gefährliche Gase austreten, wie beispielsweise Fluor, das dann giftige oder schädliche Verbindungen wie Flusssäure (HF) bilden kann. Natürlich können solche Materialien bei solchen Anwendungen verwendet werden, bei denen speziell der Rotor 2 nicht für den Einmalgebrauch ausgelegt ist.

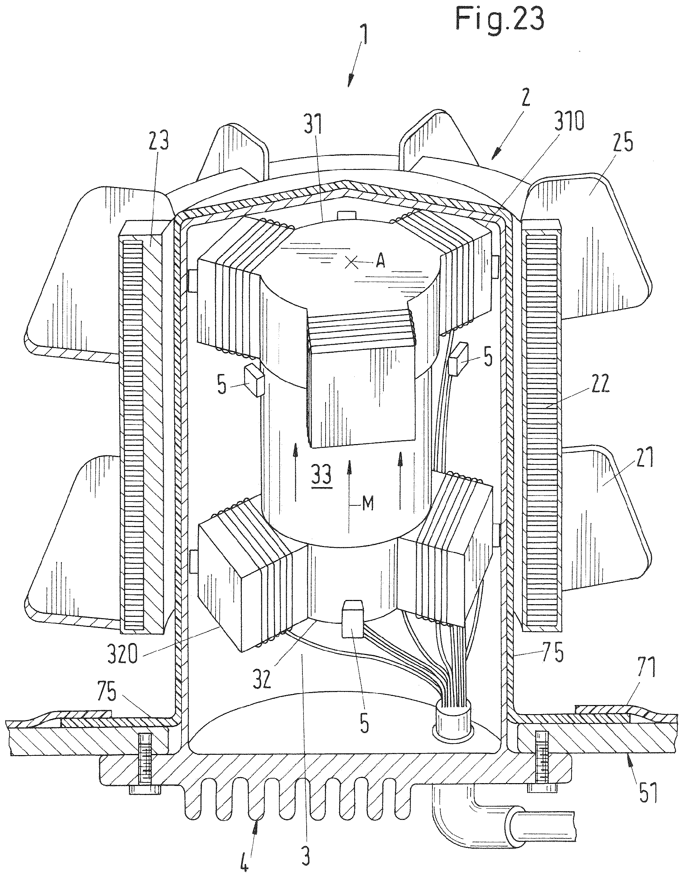

Fig. 23 zeigt in einer zu Fig. 22 analogen Darstellung eine perspektivische Schnittdarstellung eines dritten Ausführungsbeispiels eines erfindungsgemässen Drehantriebs 1, der als Pump- oder Mischvorrichtung ausgestaltet ist. Im Folgenden wird nur auf die Unterschiede zu dem vorangehend beschriebenen ersten und zweiten Ausführungsbeispiel eingegangen. Insbesondere haben die Bezugszeichen die gleiche Bedeutung wie sie bereits im Zusammenhang mit dem ersten bzw, zweiten Ausführungsbeispiel erläutert sind. Es versteht sich, dass alle vorangehend beschriebenen Varianten, Ausführungsformen und Massnahmen in gleicher Weise oder in sinngemäss gleicher Weise auch bei dem dritten Ausführungsbeispiel realisiert sein können.

Bei dem in Fig. 23 gezeigten Ausführungsbeispiel weist der Rotor 2 zwei Flügelräder 21, 25 zum Mischen der Substanzen auf, die bezüglich der axialen Richtung beabstandet zueinander angeordnet sind. Beide Flügelräder 21 und 25 sind Bestandteil des einzigen Rotors 2 und jeweils radial aussenliegend am magnetisch wirksamen Kern 22 des Rotors 2 vorgesehen.

Im Vergleich zu dem zweiten Ausführungsbeispiel weist der Stator 3 im zweiten Ausführungsbeispiel eine grössere Höhe in axialer Richtung auf. Dies kann beispielsweise dadurch realisiert werden, dass die Ausdehnung des Permanentmagneten 33 des Stators 3 in axialer Richtung vergrössert wird, sodass sich damit auch der Abstand zwischen dem unteren Statorteil 32 und dem oberen Statorteil vergrössert, oder dadurch, dass die axiale Höhe des unteren und/oder oberen Statorteils 32, 31 vergrössert wird, oder durch Kombination dieser beiden Massnahmen. Der Rotor 2 und der Stator 3 sind vorzugsweise so ausgestaltet, dass im Betriebszustand das eine Flügelrad 21 auf der gleichen Höhe (in axialer Richtung) angeordnet ist wie der untere Statorteil 32 und das andere Flügelrad 25 auf der gleichen Höhe wie der obere Statorteil 32.

Der magnetisch wirksame Kern 22 des Rotors 2 ist auch hier vorzugsweise im wesentlichen ring- bzw. zylinderförmig ausgestaltet und erstreckt sich im wesentlichen über die gesamte axiale Höhe des Rotors 2.

Im Unterschied zum zweiten Ausführungsbeispiel sind bei dem dritten Ausführungsbeispiel sowohl im Bereich des unteren Statorteils 32 Positionssensoren 5 vorgesehen als auch im Bereich des oberen Statorteils 31, sodass Verkippungen des Rotors 3 bezüglich der radialen Ebene detektiert werden können.

Bei dem dritten Ausführungsbeispiel ist es insbesondere bevorzugt, dass jede der Spulen des unteren Statorteils 32 und jede der Spulen des oberen Statorteils 31 separat ansteuerbar sind, damit der Rotor 3 bezüglich Verkippungen zur radialen Ebene (zwei Freiheitsgrade) aktiv magnetisch regelbar ist. Es versteht sich, dass die beiden Statorteile 31, 32 auch mehr als drei Spulen und Pole, insbesondere auch vier, fünf oder sechs, 5 und 6 Spulen und obere Pole 310 und untere Pole 320 aufweisen können.

Es versteht sich, dass auf dem Rotor auch mehr als zwei Flügelräder 21, 25 vorgesehen sein können.

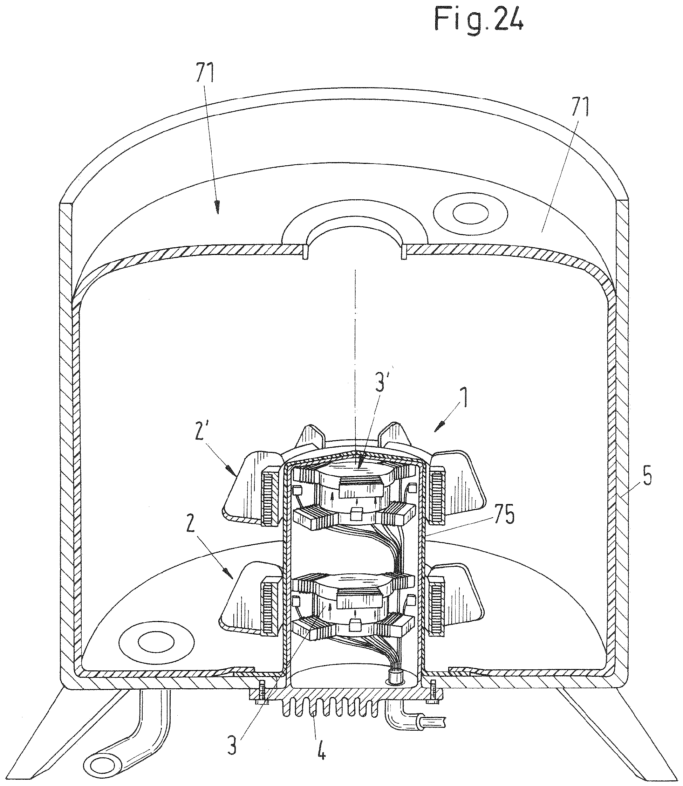

Fig. 24 zeigt in einer perspektivischen Schnittdarstellung ein viertes Ausführungsbeispiels des erfindungsgemässen Drehantriebs 1, der in eine Mischvorrichtung integriert ist. Im Folgenden wird nur auf die Unterschiede zu dem vorangehend beschriebenen ersten, zweiten und dritten Ausführungsbeispiel eingegangen. Insbesondere haben die Bezugszeichen die gleiche Bedeutung wie sie bereits im Zusammenhang mit dem ersten, zweiten bzw. dritten Ausführungsbeispiel erläutert sind. Es versteht sich, dass alle vorangehend beschriebenen Varianten, Ausführungsformen und Massnahmen in gleicher Weise oder in sinngemäss gleicher Weise auch bei dem vierten Ausführungsbeispiel realisiert sein können.

Das vierte Ausführungsbeispiel zeichnet sich dadurch aus, dass der Drehantrieb zwei Rotoren 2, 2' umfasst, die in dem Mischbehälter 71 vorgesehen sind, und von denen jeder jeweils ein Flügelrad 21, 21' zum Mischen der Substanzen umfasst. Auch hier ist jeder Rotor 2, 2' jeweils berührungslos magnetisch antreibbar und jeweils spulenfrei sowie permanentmagnetfrei ausgestaltet. Im Betriebszustand sind die beiden Rotoren 2, 2' bezüglich der axialen Richtung beabstandet und koaxial angeordnet, d. h. sie haben die gleiche Solldrehachse A.

Ferner sind zwei Statoren 3, 3' vorgesehen, von denen jeder nach einer der vorangehend beschriebenen Ausgestaltungen ausgebildet ist. Beide Statoren 3, 3' sind im Spalttopf 4 angeordnet und axial zueinander beabstandet. Der darstellungsgemäss untere Stator 3 ist dem darstellungsgemäss unteren Rotor 2 zugeordnet und bildet mit diesem einen elektromagnetischen Drehantrieb, der nach dem Prinzip des lagerlosen Motors ausgestaltet ist. Der darstellungsgemäss obere Stator 3' ist dem darstellungsgemäss unteren Rotor 2' zugeordnet und bildet mit diesem einen elektromagnetischen Drehantrieb, der nach dem Prinzip des lagerlosen Motors ausgestaltet ist.

Die beiden Rotoren 2, 2' sind ebenfalls nach einer der vorangehend beschriebenen Ausgestaltungen ausgebildet. Die beiden Rotoren 2, 2' können im Wesentlichen gleich oder auch unterschiedlich ausgestaltet sein.

Gleiches gilt für die beiden Statoren 3, 3'. Auch diese können im Wesentlich gleich oder auch unterschiedlich ausgestaltet sein, beispielsweise mit einer unterschiedlichen Anzahl von unteren oder oberen Polen 320, 310.

Der besondere Vorteil des vierten Ausführungsbeispiels liegt darin, dass hiermit deutlich mehr Mischvorgänge realisierbar sind, so ist es beispielsweise möglich, dass die beiden Rotoren 2, 2' in unterschiedlichen Richtungen und/oder mit unterschiedlichen Rotationsgeschwindigkeit drehen.

Bei den vorangehend beschriebenen Ausführungsbeispielen, Varianten und Massnahmen ist auf solche Fälle Bezug genommen, bei welchen der untere Statorteil 32 gleichviel untere Pole 320 aufweist wie der obere Statorteil 31 obere Pole 310. Das ist natürlich keinesfalls notwendigerweise so. Es sind durchaus auch Ausgestaltungen möglich, bei welchen die Anzahl der oberen Pole 310 verschieden ist von der Anzahl der unteren Pole 320.

Der Stator 3 und der Rotor 2 bilden zusammen den elektromagnetischen Drehantrieb 1, der wie bereits erläutert vorzugsweise nach dem Prinzip des lagerlosen Motors arbeitet. Beim lagerlosen Motor sind immer mindestens drei Freiheitsgerade des Rotors, nämlich seine Rotation um die Solldrehachse A und seine Position in der radialen Ebene, aktiv magnetisch regelbar. Der Freiheitsgrad der axialen Position des Rotors 3 ist beim erfindungsgemässen Drehantrieb passiv magnetisch stabilisiert, das heisst, es ist kein separates Axialmagnetlager oder mechanisches Axiallager notwendig. Dadurch wird einerseits der Rotor 2 besonders einfach und kostengünstig und andererseits lässt sich der Rotor 2 einfach vom Stator 3 und gegebenenfalls vom Spalttopf 4 trennen. Aufgrund des Fehlens axialer Lagerkomponenten kann nämlich der Rotor 2, welcher in seiner Bewegungsfreiheit bezüglich der axialen Richtung der Ausgestaltung gemäss Fig. 22 allenfalls nur über die mit dem Becher 75 verbundenen Halteelemente 752, 753 eingeschränkt ist, zusammen mit dem Becher 75 einfach axial vom Spalttopf 4 oder vom Stator 3 gezogen werden. Der magnetisch wirksame Kern 22 des Rotors 2 wird dabei bei Auslenkung in axialer Richtung durch die vom Stator ausgehenden Magnetfelder wie durch magnetische Federkräfte zurückgezogen. Dies ist in der sehr schematischen Darstellung in Fig. 28 veranschaulicht. Wird der Rotor 3 bezüglich seiner axialen Lage aus der Sollposition also aus der radialen Ebene heraus bewegt (in Fig. 28 darstellungsgemäss nach unten), so bewirkt dies passiv magnetische axiale Rückstellkräfte, dargestellt durch die Pfeile FA in axialer Richtung, welche den Rotor 2 nach dem Verschwinden der anderen äusseren Kräfte zurück in seine Sollposition bezüglich der axialen Richtung bewegen.

Dabei nehmen die Kräfte FA zunächst mit der Auslenkung zu, erreichen bei einer bestimmten Auslenkung, welche von der Geometrie des magnetisch wirksamen Kerns 22 des Rotors 2, der Geometrie des oberen Statorteils 31 und des unteren Statorteils 32, dem Abstand zwischen dem oberen Statorteil 31 und dem unteren Statorteil 32, der Geometrie und den magnetischen Eigenschaften des Permanentmagneten 33 und dem Luftspalt (dem Abstand zwischen den Statorteilen 31 und 32 einerseits und dem magnetisch wirksamen Kern 22 des Rotors 2 andererseits) abhängen, ein Maximum und nehmen dann wieder ab. Beim lagerlosen Motor für den erfindungsgemässen Drehantrieb 1 wird die Charakteristik des inhärenten axialen Passivmagnetlagers so gewählt, dass die axialen Kräfte, welche auf den Rotor 2 wirken, im ganzen Betriebsbereich unterhalb der Maximalkraft des axialen Passivmagnetlagers liegen, und dass bei solchen Anwendungen, bei welchen der Rotor 2 einfach vom Stator 3 trennbar sein soll, die Maximalkraft des axialen Passivmagnetlagers klein genug bleibt, dass sich der Rotor 2 gegebenenfalls mit dem Mischbehälter 71 einfach und ohne Werkzeug vom Spalttopf 4 bzw. dem Stator 3 trennen lässt. Dabei hat sich für Ausgestaltungen als Pump- oder Mischvorrichtung eine Maximalkraft des axialen Passivmagnetlagers von maximal 200 Newton als noch ohne Werkzeug oder Hilfsvorrichtung handhabbar herausgestellt. Bei kleineren Mischvorrichtungen, wird eine deutlich kleinere Maximalkraft des axialen Passivmagnetlagers gewählt, um das Einsetzen und Entfernen so einfach wie möglich zu gestalten. Typisch sind Werte zwischen 10 Newton und 80 Newton für Mischvorrichtungen für 50 Liter bis 1000 Liter und niederviskose Flüssigkeiten.

Für die beiden verbleibenden Freiheitsgrade, nämlich die Verkippungen des Rotors 2 relativ zur radialen Ebene, ist bei allen Ausgestaltungen mit Ausnahme derjenigen gemäss Fig. 23 ebenfalls eine passiv magnetische Stabilisierung realisierbar. Wie bereits zuvor beschrieben, wird bei solchen Ausgestaltungen die Regelung des lagerlosen Motors besonders einfach und auch die Anzahl der Leistungsverstärkerkanäle kann reduziert werden. Wie dies beim lagerlosen Motor des erfindungsgemässen Drehantriebs realisiert werden kann, ist in Fig. 27 verdeutlicht. Bei der dort dargestellten Verkippung des Rotors 2 kommt es zu passiv magnetischen Rückstellkräften, die aufgrund ihrer unterschiedlichen Richtung an der darstellungsgemäss linken und rechten Seite des Rotors 2 ein Drehmoment bewirken, welches der Verkippung entgegenwirkt, sodass der Rotor 2 auch bezüglich dieser beiden Freiheitsgrade passiv magnetisch stabilisiert ist. Diese passive Stabilisierung funktioniert aber nur, wenn gewisse geometrische Bedingungen erfüllt sind. Wenn man mit d den Innendurchmesser des Rotors 2 bezeichnet und mit h die Höhe des magnetisch wirksamen Kerns 22 des Rotors, so muss der Innendurchmesser mindestens 2,6 mal so gross sein wie die Höhe h. Es sollte somit die Bedingung d > 2.6 * h erfüllt sein, das heisst, der Innendurchmesser d sollte grösser sein als das 2,6-fache der Höhe h.

Aus diesem Grund ist es auch für die erfindungsgemässe Mischvorrichtung 1 bevorzugt, wenn der Rotor 2 bezüglich Verkippungen zur radialen Ebene (zwei Freiheitsgrade) rein passiv magnetisch stabilisiert ist, falls der Innendurchmesser des Rotors 2 mindestens 2.6 mal so gross ist wie die Höhe h des magnetisch wirksamen Kerns 22 in axialer Richtung.

Bei Ausgestaltungen der Erfindung, bei denen diese geometrische Bedingung nicht mehr erfüllt ist, wird der Rotor 2 aktiv magnetisch bezüglich dieser Verkippungen geregelt.

Bei den vorangehend beschriebenen Ausführungsbeispielen, Varianten und Massnahmen ist auf solche Fälle Bezug genommen, bei denen der aus dem Stator 3 und dem Rotor 2 gebildete elektromagnetische Drehantrieb 1 als Aussenläufer ausgestaltet ist, das heisst mit innenliegendem Stator 3 und darum herum angeordnetem Rotor 2. Es versteht sich, dass die Erfindung nicht auf solche Fälle beschränkt ist, sondern dass bei dem erfindungsgemässen Drehantrieb 1 der Rotor 2 und der Stator 3 auch einen elektromagnetischen Drehantrieb bilden können, der als Innenläufer ausgestaltet ist, also mit innenliegendem Rotor 2 und darum herum angeordneten Stator 3.

Beispielsweise bezugnehmend auf Fig. 22 oder Fig. 24 kann eine solche Ausgestaltung als Innenläufer z.B. so relisiert werden, dass der Becher 75 nicht in den Mischbehälter 71 hinein ausgerichtet ist, sondern aus dem Mischbehälter 71 heraus, als darstellungsgemäss in Fig. 22 oder Fig. 24 nach unten. Der Rotor 2 wird dann in den Innenraum des Bechers 75 gelegt und der Stator 3 wird um den Becher 75 herum angeordnet.

## Patentansprüche

1. Elektromagnetischer Drehantrieb mit einem Rotor (2), welcher berührungslos magnetisch antreibbar ist und spulenfrei ausgestaltet ist, sowie mit einem Stator (3), welcher als Lager- und Antriebsstator ausgestaltet ist, mit welchem der Rotor (2) im Betriebszustand berührungslos magnetisch um eine Solldrehachse (A) antreibbar und bezüglich des Stators (3) berührungslos magnetisch lagerbar ist, wobei der Stator (3) einen oberen Statorteil (31) mit einer Mehrzahl von ausgeprägten oberen Polen (310) zum Tragen von oberen Wicklungen (311; 312; 313; 314; 315; 316) umfasst, sowie einen unteren Statorteil (32) mit einer Mehrzahl von ausgeprägten unteren Polen (320) zum Tragen von unteren Wicklungen (321; 322; 323; 324; 325; 326), wobei der obere Statorteil (31) und der untere Statorteil (32) bezüglich der axialen Richtung beabstandet zueinander angeordnet sind und wobei zwischen dem oberen Statorteil (31) und dem unteren Statorteil (32) ein Permanentmagnet (33) vorgesehen ist, **dadurch gekennzeichnet, dass** sowohl der obere Statorteil (31) als auch der untere Statorteil (32) jeweils zum Antrieb und zur berührungslosen magnetischen Lagerung des Rotors (2) beitragen, wobei auf jedem oberen (310) und auf jedem unteren Statorpol (320) jeweils eine Spule (311, 312, 313, 314, 315, 316, 321, 322, 323, 324, 325, 326) als Wicklung angeordnet ist, wobei für jede Spule (311, 312, 313, 314, 315, 316, 321, 322, 323, 324, 325, 326) jeweils ein separater Leistungsverstärker (81) vorgesehen ist, mit welchem der Spulenstrom oder die Spulenspannung für die Spule jeweils unabhängig von den Spulenströmen oder den Spulenspannungen der anderen Spulen regelbar ist.

2. Drehantrieb nach Anspruch 1, bei welchem der Rotor (2) einen magnetisch wirksamen Kern (22) umfasst und frei von Permanentmagneten ist.

3. Drehantrieb nach einem der vorangehenden Ansprüche, bei welchem der obere Statorteil (31) oder der untere Statorteil (31) genau drei oder genau vier oder genau sechs obere (310) bzw. untere Pole (320) umfasst.

4. Drehantrieb nach einem der vorangehenden Ansprüche, bei welchem die Anzahl der oberen Pole (310) gleich der Anzahl der unteren Pole (320) ist.

5. Drehantrieb nach Anspruch 4, bei welcher der obere Statorteil (31) und der untere Statorteil (32) bezüglich der Solldrehachse (A) um einen Winkel (a) verdreht zueinander angeordnet sind, sodass in axialer Richtung gesehen die oberen Pole (310) jeweils in einer Lücke zwischen zwei benachbarten unteren Polen (320) angeordnet sind, wobei der Winkel (a) vorzugsweise 360° dividiert durch die Gesamtzahl der oberen (310) und der unteren Pole (320) beträgt.

6. Drehantrieb nach Anspruch 4, bei welchem die Anzahl der oberen Pole (310) und die Anzahl der unteren Pole (320) eine gerade Zahl ist, wobei die oberen Pole (310) und die unteren Pole (320) so angeordnet sind, dass sie sich in axialer Richtung gesehen überdecken.

7. Drehantrieb nach einem der vorangehenden Ansprüche, bei welchem der Rotor (2) mindestens ein Flügelrad (21, 25) zum Fördern von Fluiden umfasst.

8. Drehantrieb nach einem der vorangehenden Ansprüche, mit mindestens zwei separaten Rotoren (2, 2'), von denen jeder jeweils berührungslos magnetisch antreibbar ist und jeweils spulenfrei ausgestaltet ist, wobei die Rotoren (2, 2') im Betriebszustand bezüglich der axialen Richtung beabstandet zueinander und koaxial angeordnet sind, sowie mit mindestens zwei Statoren (3, 3'), von denen jeder als Lager- und Antriebsstator ausgestaltet ist, wobei jeder Stator (3, 3') jeweils einen oberen Statorteil (31) mit einer Mehrzahl von ausgeprägten oberen Polen (310) zum Tragen von oberen Wicklungen umfasst, sowie einen unteren Statorteil (32) mit einer Mehrzahl von ausgeprägten unteren Polen (320) zum Tragen von unteren Wicklungen, wobei der obere Statorteil (31) und der untere Statorteil (32) jedes Stators (3, 3') bezüglich der axialen Richtung beabstandet zueinander angeordnet sind, wobei jeweils zwischen dem oberen Statorteil (31) und dem unteren Statorteil (32) ein Permanentmagnet (33) vorgesehen ist und wobei die beiden Statoren (3, 3') im Betriebszustand bezüglich der axialen Richtung beabstandet zueinander angeordnet sind.

9. Drehantrieb nach einem der vorangehenden Ansprüche, bei welchem der magnetisch wirksame Kern (22) des Rotors (2) mehrere ausgeprägte Rotorpole (221) aufweist, welche im Betriebszustand den Polen (310, 320) des Stators (3, 3') zugewandt sind.

10. Drehantrieb nach Anspruch 9, bei welchem die Rotorpole (221) derart asymmetrisch ausgestaltet oder angeordnet sind, dass im Betriebszustand Einrastpositionen bezüglich des Stators (3) vermieden werden.

11. Drehantrieb nach Anspruch 9 oder 10, wobei der magnetisch wirksame Kern (22) des Rotors (2) ringförmig ausgestaltet ist, wobei in der Mitte bezüglich der axialen Richtung ein umlaufender Ring (222) konstanten Durchmessers ausgebildet ist und wobei die Rotorpole (221) oberhalb und unterhalb des Rings (222) vorgesehen sind.

12. Drehantrieb nach einem der vorangehenden Ansprüche, bei welcher der Permanentmagnet (33) jedes Stators (3, 3') scheibenförmig oder ringförmig ausgestaltet ist, in axialer Richtung magnetisiert ist, und jeweils den oberen Statorteil (31) mit dem unteren Statorteil (32) verbindet.

13. Drehantrieb nach einem der vorangehenden Ansprüche, ausgestaltet als Pump- oder Mischvorrichtung zum Fördern oder Mischen von fluiden Substanzen, oder als Bestandteil einer Pump-oder Mischvorrichtung zum Fördern oder Mischen von fluiden Substanzen.

## Claims

1. An electromagnetic rotary drive with a rotor (2), which can be driven magnetically without contact and which is designed without coils, and with a stator (3), which is designed as a bearing and drive stator, with which, in the operating state, the rotor (2) can be driven magnetically without contact about a desired axis of rotation (A) and can be levitated magnetically without contact with respect to the stator (3), wherein the stator (3) comprises an upper stator part (31) having a plurality of pronounced upper poles (310) for supporting upper windings (311; 312; 313; 314; 315; 316), and a lower stator part (32) having a plurality of pronounced lower poles (320) for supporting lower windings (321; 322; 323; 324; 325; 326), wherein the upper stator part (31) and the lower stator part (32) are arranged at a distance from each other with respect to the axial direction, and wherein a permanent magnet (33) is provided between the upper stator part (31) and the lower stator part (32), **characterized in that** both the upper stator part (31) and the lower stator part (32) contribute respectively to the drive and to the contactless magnetic levitation of the rotor (2), wherein on each upper stator pole (310) and on each lower stator pole (320) in each case one coil (311, 312, 313, 314, 315, 316, 321, 322, 323, 324, 325, 326) is arranged as a winding, wherein a separate power amplifier (81) is provided in each case for each coil (311, 312, 313, 314, 315, 316, 321, 322, 323, 324, 325, 326), by means of which the coil current or the coil voltage for the coil can be regulated in each case independently of the coil currents or the coil voltages of the other coils.

2. The rotary drive according to claim 1, in which the rotor (2) comprises a magnetically effective core (22) and is free of permanent magnets.

3. The rotary drive according to anyone of the preceding claims, in which the upper stator part (31) or the lower stator part (31) comprises exactly three or exactly four or exactly six upper (310) or lower poles (320), respectively.

4. The rotary drive according to anyone of the preceding claims, in which the number of upper poles (310) is equal to the number of lower poles (320).

5. The rotary drive according to claim 4, in which the upper stator part (31) and the lower stator part (32) are arranged rotated relative to one another by an angle (α) with respect to the desired axis of rotation (A), so that, viewed in the axial direction, the upper poles (310) are each arranged in a gap between two adjacent lower poles (320), wherein the angle (α) preferably is 360° divided by the total number of upper (310) and lower poles (320).

6. The rotary drive according to claim 4, in which the number of upper poles (310) and the number of lower poles (320) is an even number, wherein the upper poles (310) and the lower poles (320) are arranged such that they overlap as viewed in the axial direction.

7. The rotary drive according to anyone of the preceding claims, in which the rotor (2) comprises at least one impeller (21, 25) for conveying fluids.

8. The rotary drive according to anyone of the preceding claims, with at least two separate rotors (2, 2'), each of which can be driven magnetically without contact and each of which is designed without coils, wherein, in the operating state, the rotors (2, 2') are arranged at a distance from each other and coaxially with respect to the axial direction, and with at least two stators (3, 3'), each of which is designed as a bearing and drive stator, wherein each stator (3, 3') comprises in each case an upper stator part (31) having a plurality of pronounced upper poles (310) for supporting upper windings, and a lower stator part (32) having a plurality of pronounced lower poles (320) for supporting lower windings, wherein the upper stator part (31) and the lower stator part (32) of each stator (3, 3') are arranged at a distance from each other with respect to the axial direction, wherein a permanent magnet (33) is provided in each case between the upper stator part (31) and the lower stator part (32) and wherein, in the operating state, the two stators (3, 3') are arranged at a distance from each other with respect to the axial direction.

9. The rotary drive according to anyone of the preceding claims, in which the magnetically effective core (22) of the rotor (2) has several pronounced rotor poles (221) which, in the operating state, face the poles (310, 320) of the stator (3, 3').

10. The rotary drive according to claim 9, in which the rotor poles (221) are asymmetrically designed or arranged in such a way that engagement positions with respect to the stator (3) are avoided in the operating state.

11. The rotary drive according to claim 9 or 10, wherein the magnetically effective core (22) of the rotor (2) is designed in the form of a ring, wherein a circumferential ring (222) of constant diameter is formed in the center with respect to the axial direction and wherein the rotor poles (221) are provided above and below the ring (222).

12. The rotary drive according to anyone of the preceding claims, in which the permanent magnet (33) of each stator (3, 3') is designed in the form of a disk or a ring, is magnetized in the axial direction, and in each case connects the upper stator part (31) to the lower stator part (32).

13. The rotary drive according to anyone of the preceding claims, designed as a pumping or mixing device for conveying or mixing fluid substances, or as a component of a pumping or mixing device for conveying or mixing fluid substances.

## Revendications

1. Un entraînement rotatif électromagnétique avec un rotor (2), qui peut être entraîné magnétiquement sans contact et qui est conçu sans bobines, ainsi qu'avec un stator (3), qui est conçu comme un stator de palier et d'entraînement, avec lequel le rotor (2), en état de fonctionnement, peut être entraîné magnétiquement sans contact autour d'un axe de rotation de consigne (A) et peut être mis en lévitation magnétique sans contact par rapport au stator (3), dans lequel le stator (3) comprend une partie du stator supérieure (31) ayant une pluralité de pôles supérieurs prononcés (310) pour supporter des enroulements supérieurs (311; 312; 313; 314; 315; 316), ainsi qu'une partie du stator inférieure (32) ayant une pluralité de pôles inférieurs prononcés (320) pour supporter des enroulements inférieurs (321; 322; 323; 324; 325; 326), dans lequel la partie du stator supérieure (31) et la partie du stator inférieure (32) sont disposées à une certaine distance l'une de l'autre par rapport à la direction axiale, et dans lequel un aimant permanent (33) est prévu entre la partie du stator supérieure (31) et la partie du stator inférieure (32), **caractérisé en ce que** la partie du stator supérieure (31) ainsi que la partie du stator inférieure (32) contribuent respectivement à l'entraînement et à la lévitation magnétique sans contact du rotor (2), dans lequel sur chaque pôle supérieur du stator (310) et sur chaque pôle inférieur du stator (320), une bobine (311, 312, 313, 314, 315, 316, 321, 322, 323, 324, 325, 326) est disposée en tant qu'enroulement, dans lequel un amplificateur de puissance séparé (81) est prévu pour chaque bobine (311, 312, 313, 314, 315, 316, 321, 322, 323, 324, 325, 326), au moyen duquel le courant de bobine ou la tension de bobine pour la bobine peut être régulé indépendamment des courants de bobine ou des tensions de bobine des autres bobines.

2. L'entraînement rotatif selon la revendication 1, dans lequel le rotor (2) comprend un noyau (22) à effet magnétique et est exempt d'aimants permanents.

3. L'entraînement rotatif selon l'une des revendications précédentes, dans lequel la partie du stator supérieure (31) ou la partie du stator inférieure (31) comprend exactement trois ou exactement quatre ou exactement six pôles supérieurs (310) ou inférieurs (320), respectivement.

4. L'entraînement rotatif selon l'une des revendications précédentes, dans lequel le nombre de pôles supérieurs (310) est égal au nombre de pôles inférieurs (320).

5. L'entraînement rotatif selon la revendication 4, dans lequel la partie du stator supérieure (31) et la partie du stator inférieure (32) sont disposées en étant tournées l'une par rapport à l'autre d'un angle (α) par rapport à l'axe de rotation de consigne (A), de sorte que, vu dans la direction axiale, les pôles supérieurs (310) sont chacun disposés dans un espace entre deux pôles inférieurs (320) adjacents, l'angle (a) étant de préférence de 360° divisé par le nombre total de pôles supérieurs (310) et inférieurs (320).

6. L'entraînement rotatif selon la revendication 4, dans lequel le nombre de pôles supérieurs (310) et le nombre de pôles inférieurs (320) est un nombre pair, dans lequel les pôles supérieurs (310) et les pôles inférieurs (320) sont disposés de telle sorte qu'ils se chevauchent lorsqu'ils sont vus dans la direction axiale.

7. L'entraînement rotatif selon l'une des revendications précédentes, dans lequel le rotor (2) comprend au moins une roue à aubes (21, 25) pour convoyer des fluides.

8. L'entraînement rotatif selon l'une des revendications précédentes, avec au moins deux rotors séparés (2, 2'), dont chacun peut être entraîné magnétiquement sans contact et est conçu sans bobines, dans lequel les rotors (2, 2'), en état de fonctionnement, sont disposés à une certaine distance l'un de l'autre et coaxialement par rapport à la direction axiale, ainsi qu'avec au moins deux stators (3, 3'), dont chacun est conçu comme un stator de palier et d'entraînement, dans lequel chaque stator (3, 3') comprend respectivement une partie du stator supérieure (31) ayant une pluralité de pôles supérieurs prononcés (310) pour supporter des enroulements supérieurs, ainsi qu'une partie du stator inférieure (32) ayant une pluralité de pôles inférieurs prononcés (320) pour supporter des enroulements inférieurs, dans lequel la partie du stator supérieure (31) et la partie du stator inférieure (32) de chaque stator (3, 3') sont disposées à une certaine distance l'une de l'autre par rapport à la direction axiale, dans lequel un aimant permanent (33) est prévu respectivement entre la partie du stator supérieure (31) et la partie du stator inférieure (32) et dans lequel les deux stators (3, 3') sont disposées à une certaine distance l'une de l'autre par rapport à la direction axiale en état de fonctionnement.

9. L'entraînement rotatif selon l'une des revendications précédentes, dans lequel le noyau (22) à effet magnétique du rotor (2) présente plusieurs pôles de rotor (221) prononcés qui, en état de fonctionnement, sont tournés vers les pôles (310, 320) du stator (3, 3').

10. L'entraînement rotatif selon la revendication 9, dans lequel les pôles de rotor (221) sont conçus ou disposés de manière asymétrique de telle sorte que des positions d'engagement par rapport au stator (3) sont évitées en état de fonctionnement.

11. L'entraînement rotatif selon la revendication 9 ou 10, dans lequel le noyau (22) à effet magnétique du rotor (2) est conçu en forme d'anneau, dans lequel un anneau périphérique (222) de diamètre constant est formé au centre par rapport à la direction axiale et dans lequel les pôles du rotor (221) sont prévus au-dessus de et en dessous de l'anneau (222).

12. L'entraînement rotatif selon l'une des revendications précédentes, dans lequel l'aimant permanent (33) de chaque stator (3, 3') est conçu en forme de disque ou d'anneau, est magnétisé dans la direction axiale, et relie respectivement la partie du stator supérieure (31) à la partie du stator inférieure (32).

13. L'entraînement rotatif selon l'une des revendications précédentes, conçu comme dispositif de pompage ou de mélange pour convoyer ou mélanger des substances fluides, ou comme composant d'un dispositif de pompage ou de mélange pour convoyer ou mélanger des substances fluides.
